(19)

Europäisches
Patentamt
European
Patent Office
Office européen
des brevets

(11) **EP 4 620 437 A1**

(12) **EUROPEAN PATENT APPLICATION**

(43) Date of publication:
24.09.2025 Bulletin 2025/39

(51) International Patent Classification (IPC):
**A61F 5/01** (2006.01)

(21) Application number: 24165234.6

(22) Date of filing: **21.03.2024**

(52) Cooperative Patent Classification (CPC):
**A61F 5/013**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Universität Heidelberg**
**69117 Heidelberg (DE)**

(72) Inventors:
• **MISSIROLI, Francesco**
**69120 Heidelberg (DE)**
• **MASIA, Lorenzo**
**69120 Heidelberg (DE)**

(74) Representative: **Müller-Boré & Partner**
**Patentanwälte PartG mbB**
**Friedenheimer Brücke 21**
**80639 München (DE)**

(54) **USER-WEARABLE ORTHOSIS, METHOD FOR CONTROL THEREOF, AND NON-TRANSITORY COMPUTER READABLE STORAGE MEDIUM**

(57) The disclosure relates to a user-wearable orthosis for assisting a user motion of a joint of a user, comprising a proximal anchor unit mountable on or proximal to the joint, a distal anchor unit mountable on a limb attached to the joint, a movement prediction unit configured to at least partially predict the user motion of the joint, and an anchor point element adjustment unit. The distal anchor unit comprises an anchor point element moveable at least partially circumferentially around the limb of the user, wherein the proximal anchor unit is connectable to the anchor point element via a force transfer element, wherein the user-wearable orthosis is configured to exert a pulling force on the force transfer element to assist the user motion. The anchor point element adjustment unit is configured to control a circumferential position of the anchor point element around the limb based on the at least partially predicted user motion of the joint. The disclosure further relates to a corresponding method for controlling a user-wearable orthosis and a non-transitory computer-readable storage medium.

Figure 2

EP 4 620 437 A1

**Description**

The present invention relates to a user-wearable orthosis for assisting a user motion of a joint of a user, a method for controlling a user-wearable orthosis for assisting a user motion of a joint of a user, and a corresponding non-transitory computer readable storage medium.

The invention lies in the field of user-wearable orthoses, and in particular in the field of user-wearable orthoses for user motion assistance. Specifically, users may be restricted in the movement of one or more of their joints for a large number of reasons. For example, users may suffer from injury or illness that impacts their ability to move said one or more joints as desired. Similarly, other factors may impact and/or reduce a user's ability to move their joints, such as factors linked to the specific desired movement (e.g., moving a particularly heavy load or performing particularly strenuous tasks) and/or physiological factors (e.g., user fatigue). To aid in performing such movements or restoring the ability to perform said movements, different types of mechanical devices have previously been developed.

In particular, rigid exoskeletons have been developed to assist user motion. However, such rigid exoskeletons typically rely on the provision of passive, rigid links (such as spring loaded links) configured to work in parallel to a user joint, such as a shoulder joint. The inventors realised during the conception of the present invention that, while avoiding the use of electrical actuation, such rigid exoskeletons relying on passive links suffer from a significant number of drawbacks, such as: increased system complexity, increased weight and size (reducing, in turn, portability and user comfort), lack of assistive force modulation, reduced comfort, and increased system cost. For example, typical rigid exoskeletons have a weight of between 6 and 8 kg, and thus impose a significant burden on the wearer/user. The inventors further realised that the use of rigid links, e.g., passive rigid links, leads to an unnatural alteration of the respective joint's physiological motion, causing unexpected overloads and parasitic torques at the articulation.

Some rigid exoskeletons also implement the use of active actuation via either pneumatic or electrical actuation to enable support along one degree of freedom. However, such exoskeletons require the provision of the necessary hardware. For example, a pneumatic rigid exoskeleton requires the provision of at least one compressor stage. Furthermore, a controllability of such pneumatic rigid exoskeletons is limited due to the non-linearity of the inflatable/deflatable pneumatic elements, e.g., inflatable sacks.

It was further recognized by the inventors during the conception of the present invention that typical assistive technology is capable of assisting a motion of a joint in only one degree of freedom, e.g., only for flexion/extension or only for abduction/adduction. However, this may lead to inadequate assistance to user motion and/or cause deviation from the normal physiological motion of the user and/or lead to injury of the user and/or lead to abandonment of the assistive technology.

The present invention addresses and overcomes the above-discussed drawbacks. It is therefore an object of the invention to provide means for assisting a user motion of a joint of a user having improved operational characteristics.

Said object is solved by a user-wearable orthosis for assisting a user motion of a joint of a user, a method for controlling a user-wearable orthosis for assisting a user motion of a joint of a user, and a non-transitory computer readable storage medium, as recited in the independent claims. In particular, the invention is defined by the independent claims, while preferred embodiments form the subject of the dependent claims.

An aspect of the disclosure relates to a user-wearable orthosis for assisting a user motion of a joint of a user. The user-wearable orthosis may in particular be a soft exosuit.

"User-wearable" may be understood herein as being configured to be, at least partially, worn directly or indirectly on the user, preferentially directly or indirectly on a skin of the user. For example, the user-wearable orthosis may be configured to be worn at least partially in direct contact with the skin of a user. Alternatively, the user-wearable orthosis may be configured to be at least partially worn proximal to the user, preferably proximal to the skin of the user, wherein one or more intermediate elements may be positionable between the user-wearable orthosis and the skin of the user. The user-wearable orthosis may comprise the one or more intermediate elements. The one or more intermediate elements may, for example, comprise one or more clothing items worn by the user.

The one or more intermediate elements may comprise at least one cushioning element, whereby for example a comfort of wear of the user may be improved and/or a chance of injuries of the user may be reduced and/or prevented. Alternatively, or additionally, the one or more intermediate elements may comprise at least one conforming element configured to at least partially conform to a shape of the user-wearable orthosis and/or to at least partially conform to a shape of the user, whereby a better fit and/or higher comfort of the user-wearable orthosis may be achieved.

In addition, the user-wearable orthosis and/or the one or more intermediate elements may be configured to prevent and/or reduce a relative motion between the user-wearable orthosis and the user, such as a slipping motion of the user-wearable orthosis relative to the user and/or to the skin of the user. Specifically, it may therefore be possible to further improve the assistance of the user motion, for example by improved force transmission properties. This may be, at least in part, achieved, merely as an example, by increasing a friction between the user-wearable orthosis and/or the one or more intermediate elements on the one hand, and the user, e.g., the skin of the user, on the other hand. Alternatively, or additionally, this may also be, at least partially, achieved by arrangement of the user-wearable orthosis at one or more predetermined locations on the user.

The one or more intermediate elements may comprise a user-wearable harness. The user-wearable orthosis and/or parts of the user-wearable orthosis may be fixable to and/or mountable on the user-wearable harness. The user-wearable harness may be an integral harness or comprise one or more harness elements. The one or more harness elements may be configured to be worn on one or more parts of the user. At least two harness elements may be, preferentially physically and/or releasably, connectable to one another, such as (merely as one of many possible examples) by belts and/or clips. The one or more harness elements may comprise at least one shoulder harness element configured to be worn at least partially on a shoulder and/or on a shoulder joint of the user. The one or more harness elements may comprise at least one torso harness element configured to be worn at least partially on a torso and/or waist of the user. The one or more harness elements may comprise at least one arm harness element configured to be worn at least partially on an upper arm and/or at least partially on a lower arm of the user. In some examples, the arm harness element may comprise only an upper part configured to be worn on the upper arm. In other examples, the arm harness element may comprise an upper part and a lower part configured to be worn on the lower arm. The lower part may be useful to improve friction, e.g. to avoid squeezing the arm of the user too much.

In a particular example, the user-wearable harness may comprise three harness elements: a torso harness element, a shoulder harness element and an arm harness element. The torso harness element may hold most of the weight of the orthosis and may discharge this weight on the spine of the user by being placed on the torso of the user. The shoulder harness element may be the part discharging the counteracting forces of the orthosis to the user and providing a solid base to transmit the force to the user. The arm harness element may be the main part delivering assistive force to the user limb. The three harness elements may be kept separate to adapt to different size and shapes of the user body and improve the wearability of the device.

Please note that the user-wearable orthosis is not restricted to the above exemplary harness elements. Therefore more, less, and/or different harness elements may be provided according to the desired assistance to be provided to a desired joint of the user. Different parts of the user-wearable orthosis may be fixable to and/or mountable on different harness elements. The user-wearable harness may be a textile harness. The one or more harness elements may be one or more textile harness elements.

Furthermore, at least one surface and/or at least part of a surface of the user-wearable orthosis may be shaped to at least partially conform to the shape of the user at a position of wear of the user-wearable orthosis on the user. In particular, it may therefore be possible to enable an individualised fit of the user-wearable orthosis for the user. Alternatively, at least one surface and/or at least part of a surface of the user-wearable orthosis may be shaped according to population statistics, such as according to one or more of average body shapes, size, age, gender, etc. Such an approach may for example enable a manufacture of the user-wearable orthosis even if individual characteristics and/or parameters and/or shapes of a specific user are not known prior to manufacture thereof. Furthermore, this, e.g., in combination with a provision of at least one cushioning element and/or at least one conforming element, may allow for mass manufacture of the user-wearable orthosis.

The user may in particular be a human user. However, the user-wearable orthosis is not limited thereto, and other users such as for example an animal user may also be possible.

A joint of a user may connect two or more structures, such as for example bones, of a skeletal system of the user. The user-wearable orthosis may be configured to assist the user motion by generating a direct and/or indirect force, such as a pulling force, on at least one structure to cause a motion, such as a bending motion, of the joint of the user. The user-wearable orthosis may be configured to assist a user motion of one or more joints of the user simultaneously and/or sequentially.

Furthermore, "assisting a user motion of a joint" and/or "assistance of a user motion of a joint" may be understood within the scope of this disclosure to relate to physical and/or mechanical assistance, such as through exertion of one or more forces, provided to the user in order to facilitate and/or support the user motion. Merely as an example, said one or more forces may be exerted on an upper arm of the user to assist an arm raising motion of the shoulder joint of the user. As another possible example, one or more forces may be exerted on an upper thigh of the user to assist a leg raising motion of a hip joint of the user. However, other types of user motions may alternatively or additionally also be assisted.

The user motion of the joint of the user may for example be one or more of a joint flexion, a joint extension, a joint abduction, and a joint adduction. The user motion may comprise a plurality of joint motions. For example, the user motion may comprise a sequential combination of joint motions, e.g., of the above discussed joint motions, such as for example a joint extension followed by a joint abduction, or a joint abduction followed by a further joint abduction. The user-wearable orthosis may be configured to assist the sequential combination of joint motions. However, other motions may also be possible, and may contain more and/or different joint motion combinations. For example, the user motion may comprise a sequence of at least two joint motions, preferably at least two different joint motions, further preferably at least two different joint motions along at least two different planes of motion.

The user motion of the joint may be a user motion in at least one plane of the user. For example, the user motion may be a user motion in a frontal plane of the user, a user motion in a sagittal plane of the user, and/or a user motion having motion components in and/or along and/or parallel to both the frontal plane and the sagittal plane.

The user-wearable orthosis comprises a proximal anchor unit mountable on or proximal to the joint. The proximal anchor unit may be connectable to at least one force transfer element. In particular, the proximal anchor unit may be configured to be connectable to at least one force transfer element to at least partially guide said at least one force transfer element between a distal anchor unit and an actuator connected to the at least one force transfer element. The proximal anchor unit may be mountable on the integral harness or one of the harness elements. For example, the proximal anchor unit may be mountable on a shoulder harness element.

The at least one force transfer element may comprise one or more tendons, one or more cables, one or more strips, and/or one or more ribbons. However, the at least one force transfer element is not restricted to the above list. Instead, the at least one force transfer element may comprise any element suitable for having a pulling force exerted thereon in order to assist the user motion. Examples of further elements capable of transmitting tensile force may be found in "Soft Robotic Suits: State of the Art, Core Technologies, and Open Challenges" by M. Xiloyannis et al., IEEE Transactions on Robotics ( Volume: 38, Issue: 3, June 2022).

The at least one force transfer element may further comprise at least one Bowden sheath, wherein the at least one force transfer element is configured to extend at least partially within the at least one Bowden sheath. Preferentially, each force transfer element comprises exactly one or two corresponding Bowden sheath(s). The user-wearable orthosis may comprise the at least one force transfer element. Preferentially, the user-wearable orthosis may comprise one force transfer element.

The user-wearable orthosis further comprises a distal anchor unit mountable on a limb attached to the joint. The distal anchor unit may be connectable to the at least one force transfer element. The distal anchor unit may be mountable on the integral harness or one of the harness elements. For example, the distal anchor unit may be mountable on an arm harness element, e.g. on an upper part of the arm harness element. The limb may be a limb of the user, wherein assisting the user motion comprises assisting a movement of the limb. For example, the limb may be an arm, preferentially an upper arm, or a leg, preferentially a thigh, of the user.

The distal anchor unit comprises an anchor point element moveable at least partially circumferentially, preferentially along a circumferential direction, around the limb of the user. In other words, the distal anchor unit comprises the anchor point element moveable at least partially along a circumferential direction around the limb of the user. The limb of the user may extend substantially along a limb axis. For example, an upper arm of the user may extend substantially along the limb axis (e.g., an upper arm axis), wherein the limb axis (e.g., the upper arm axis) extends substantially along an axis of the humerus bone of the upper arm. The circumferential direction may further be a direction along a plane substantially perpendicular to the limb, preferentially substantially perpendicular to a primary extension dimension and/or the limb axis of the limb.

The distal anchor unit may be shaped depending on the joint. In some cases, the distal anchor unit may be configured to completely surround the limb of the user along the circumferential direction. In other cases, the distal anchor unit may be configured to at least partially surround the limb of the user along the circumferential direction. The distal anchor unit may extend around the circumferential direction within a circumferential range of at least approx. 90°, preferentially at least approx. 135°, further preferentially at least approx. 180°. In particular, by providing such a distal anchor unit, a full range of motion of the user motion may be assisted. The distal anchor unit may extend around the circumferential direction within a circumferential range of at most approx. 305°, preferentially at most approx. 270°, further preferentially at most approx. 225°. In particular, by providing such a distal anchor unit, a full range of motion of the user motion may be assisted, while also improving an ease of use and ease of donning/doffing of the user-wearable orthosis. The distal anchor unit and/or the circumferential range of the distal anchor unit may in particular be configured/selected such that, preferentially in a mounted state of the user-wearable orthosis on the user, the distal anchor unit and/or the circumferential range of the distal anchor unit intersect at least the frontal plane of the user and/or the sagittal plane of the user.

In one example, the distal anchor unit may extend around the circumferential direction within a circumferential range of about 180°. In another example, the distal anchor unit may extend around the circumferential direction within a circumferential range of about 270°.

The mounted state of the user-wearable orthosis on the user may be a state in which the user-wearable orthosis is worn by the user such that the user-wearable orthosis is capable of assisting the user motion.

The proximal anchor unit is connectable to the anchor point element via at least one force transfer element. In particular, the at least one force transfer element may be fixedly or releasably connectable to the anchor point element. For example, the anchor point element may be connectable to a tendon, wherein the tendon is further connectable to the proximal anchor unit.

The user-wearable orthosis is configured to exert a pulling force on the force transfer element to assist the user motion. For example, the user-wearable orthosis may comprise a motor or actuator to exert the pulling force on the force transfer element. The user-wearable orthosis may be configured to controllably exert the pulling force on the force transfer element in accordance with the user motion. In particular, it may therefore be possible to adjust the exerted pulling force according to the desired user motion in order to allow an optimal assistance of said user motion.

In some examples, the orthosis may comprise a plurality of anchor point elements. For instance, if the joint is the hip, the orthosis may comprise two anchor point elements, e.g. one on the hip and one on the thigh, and two motors, wherein one motor could act as gravity compensator while the second one could be used to synchronously reconfigure the location of the anchor point elements.

The user-wearable orthosis further comprises a motion prediction unit configured to at least partially predict the user motion of the joint. In particular, the motion prediction unit may be configured to predict the user motion in and/or along at least one plane relative to the user, such as in the frontal plane and/or in the sagittal plane of the user. The motion prediction may be continuous all over the movement of the user. The motion prediction may be related to the elevation angle $\alpha$ (discussed below). This is done to penalise the rotation in the anchor point with high elevation angles and improve the stability of the system.

The user-wearable orthosis further comprises an anchor point element adjustment unit. The anchor point element adjustment unit is configured to control a circumferential position of the anchor point element around the limb based on the at least partially predicted user motion of the joint. In particular, the anchor point element adjustment unit may be configured to receive the at least partially predicted user motion from the motion prediction unit and convert said at least partially predicted user motion into a localised position along the circumferential direction around the limb of the user. The localised position may correspond to an angular position around the limb of the user. In particular, the localised position and the at least partially predicted user motion may lie in a common plane. The anchor point element adjustment unit may then be configured to adjust the circumferential position of the anchor point element to coincide with the localised position. However, the user-wearable orthosis is not limited to such an embodiment. For example, the motion prediction unit may be configured to convert the at least partially predicted user motion into the localised position along the circumferential direction around the limb of the user, wherein the anchor point element adjustment unit is configured to receive said localised position from the motion prediction unit. The anchor point element adjustment unit may comprise an adjustment PID position controller configured to control a circumferential position of the anchor point element around the limb based on the at least partially predicted user motion of the joint and/or the localised position.

In particular, the anchor point element adjustment unit may be configured to dynamically and/or variably control the circumferential position of the anchor point element around the limb. Specifically, it may therefore be possible to always provide the optimal motion assistance to the user even for variable and/or complex user motions.

Merely as an example of operation, the motion prediction unit may determine and/or predict the user motion in the sagittal plane of the user, such as when the user attempts to lift/flex the limb in the sagittal plane. Based on said determined and/or predicted user motion, the anchor point element adjustment unit and/or the motion prediction unit may be configured to correspondingly determine the localised position as a position in the sagittal plane of the user and along the circumferential direction, e.g., for when the limb is an arm, a position on a biceps side of the arm. Consequently, the anchor point element adjustment unit may adjust the circumferential position of the anchor point element to the determined localised position, thereby allowing the provision of a very efficient and optimized assistance of the user motion.

Merely as a further example of operation, the motion prediction unit may determine and/or predict the user motion in the frontal plane of the user, such as when the user attempts to lift/abduct the limb in the frontal plane. Based on said determined and/or predicted user motion, the anchor point element adjustment unit and/or the motion prediction unit may be configured to correspondingly determine the localised position as a position in the frontal plane of the user and along the circumferential direction, e.g., for when the limb is an arm, a position on a lateral, outer side of the arm. Consequently, the anchor point element adjustment unit may adjust the circumferential position of the anchor point element to the determined localised position, thereby allowing the provision of a very efficient and optimized assistance of the user motion.

In particular, by providing a user-wearable orthosis, as disclosed herein, it is possible to enable provision of a fully-portable, powered, user-wearable, robotic soft suit for user motion assistance, such as during performing strenuous tasks in the working environment or during rehabilitation. In particular, the user-wearable orthosis described herein may be a light-weight wearable system or an exosuit that may be tendon-driven and reconfigurable. In particular, the user-wearable orthosis may be a soft exosuit.

In particular, the user-wearable orthosis may therefore be configured to assist the user in two degrees of freedom of the joint. This is in contrast to the currently available systems, which only allow support in one degree of freedom. In other words, compared to previously introduced devices for joint (e.g., shoulder joint) support, the present invention may be configured as a soft system (exosuit) to support two degrees of freedom using a single tendon-driven actuating unit, exploiting motion prediction that is computed during, for example, an initial phase of movement of the user motion. At least some of the technical advantages achieved therewith include increased system efficiency and a reduction in overall device weight, enabling full assistance modulation at the joint of the user.

The disclosed system/orthosis may therefore offer continuous support of the user motion in the frontal plane of the user and/or the sagittal plane of the user. Hence, the presently disclosed system may enable support of the user motion in two degrees of freedom: flexion/extension and abduction/adduction.

The user-wearable orthosis may further provide assistance proportional to a 3D joint (e.g., shoulder joint) and/or limb (e.g., arm) orientation of the user. Furthermore, the user-wearable orthosis may provide accurate assistance compensating the gravity of the limb (e.g., arm) of the user taking into account orientation and centre of mass allowing a more physiological motion in contrast to typical, known passive devices.

In addition, the user-wearable orthosis enables the implementation of an underactuated soft system, which drastically reduces the mass, size, cost, complexity, and power requirements. Indeed, the user-wearable orthosis may add only negligible mass to the user's body during wear and operation of the user-wearable orthosis, which significantly improves, for example, comfort and wearability.

Furthermore, the present user-wearable orthosis may easily be adapted to different body shapes and sizes, wherein the user-wearable orthosis may be made of modular and precisely adjustable components, such as for example a custom size user-wearable orthosis comprising actuation and control units, and textile garments wrapped around the arm of the user. The user may wear each component separately. A connection between modules and/or components of the user-wearable orthosis may happen through appropriately designed anchor points and/or connection points able to adapt to different arm sizes. In addition, assembly modularity makes replacement of single components in case of damage easy, rapid, and cost-effectively.

Exemplarily, the user-wearable orthosis may be configured as an assistive device for an overhead workload, e.g. at least for elevation angles up to 90°.

The radial height of the anchor point element and the height of the cantilever over the shoulder may be determined using a holistic approach that considers both biomechanical effectiveness and user experience, ensuring that the orthosis remains functional and comfortable. In a particular example, the height of the cantilever may be in a range between about 1 cm and about 20 cm and the radial height may be from about 0 cm to 8 or 10 cm.

Exemplarily, the distal anchor unit may be configured to be as close as possible with respect to the elbow joint to optimize the transfer force and the range of motion of the device. The proximal anchor unit may be configured to be positioned at the acromion, however it may also be placed closer to the head of the user or on the back of the user.

The user-wearable orthosis and/or the distal anchor unit and/or the anchor point element and/or the anchor point element adjustment unit may further comprise a locking device. The locking device may be configured to be switchable between a locked state and an unlocked state. The locking device may be configured to prevent movement of the anchor point element around the limb in the locked state. Specifically, unintentional movements of the anchor point element, such as during exertion of the pulling force, can efficiently be prevented and/or reduced. The locking device may be configured to allow movement of the anchor point element around the limb in the unlocked state.

In particular, the locking device may be configured to be switchable from the unlocked state into the locked state by exerting of the pulling force. The locking device may be further configured to be switchable from the locked state into the unlocked state when exerting the pulling force is ceased. In particular, the locking device may be configured to remain in the unlocked state as long as no pulling force is exerted.

The locking device may comprise at least one first engagement element and at least one second engagement element, wherein at least one first engagement element and at least one second engagement elements are configured to enter into engagement with one another in the locked state. The at least one first engagement element may be comprised by the distal anchor unit, such as by the guidance element and/or the guide rail. The at least one second engagement element may be comprised by the anchor point element. The at least one first engagement element and the at least one second engagement element may comprise one or more of at least one geared cog and/or at least one geared rail. However, the locking device is not limited to such a configuration, and other means of preventing movement of the anchor point element around the limb in the locked state may be implemented, such as for example by the generation of pressure between the distal anchor unit and/or the guidance element and/or the guide rail, and the anchor point element. For example, the locking device may be configured as a braking unit, e.g., an arresting brake unit.

The distal anchor unit may further comprise a guidance element configured to at least partially circumferentially surround the limb of the user. The guidance element may, for example, comprise a guide rail and/or a guide groove. However, the guidance element is not limited thereto, and may for example comprise one or more guide rails and/or one or more guide grooves. In particular, the anchor point element may be movable along the guidance element. The guidance element may be configured to allow the anchor point element to move circumferentially around the limb and/or along the circumferential direction within the circumferential range, while preventing and/or restricting movement of the anchor point element in other directions. In particular, the anchor point element adjustment unit may be configured to control, e.g., variably control, a position of the anchor point element along the guidance element.

As an example, the guidance element may comprise a base plate configured to at least partially surround the limb of the user in the circumferential direction. The base plate may have a base plate length along the circumferential direction around the limb of the user, a base plate width along a width direction perpendicular to the base plate length and/or parallel to the limb axis, and a base plate thickness substantially parallel to a radial direction relative to the limb axis. The base plate length may in particular be chosen according to the circumferential range. Exemplarily, the base plate length may be about 20 cm, the base plate width may be about 3 cm and the base plate thickness may be 0.5 cm.

The base plate may be configured to be mountable on a harness element, e.g., an arm harness element. The base plate may further comprise one or more through-holes along the radial direction, wherein said through-holes may be configured to reduce a weight of the base plate. For example, the base plate may be configured to have a substantially honeycomb-like structure. Thereby, a weight reduction of the base plate may be achieved, while maintaining good structural rigidity. The through-holes may also be used for sewing the base plate to the harness element.

The guidance element may further comprise at least one guide rail, wherein each guide rail may comprise at least a first guide rail element and a second guide rail element.

The first guide rail element may be a rib element extending along an outer surface of the base plate. The outer surface of the base plate may in particular be an outer surface of the base plate in the radial direction. The rib element may extend along the base plate length. The rib element may further have a rib element width along the width direction and a rib element height along the radial direction from the base plate. Exemplarily, the rib element width may be about 1.2 cm and the rib element height may be about 1 cm.

Therefore, the rib element may comprise an upper rib surface, for example substantially parallel to the outer surface of the base plate, a first rib side surface, and a second rib side surface, wherein each rib side surface extends between the upper rib surface and outer surface of the base plate on opposite sides of the rib element along the width direction. The rib element may further comprise one or more through-holes, for example along the width direction, wherein said through-holes may be configured to reduce a weight of the rib element.

The rib element may be configured to at least partially guide the anchor point element along the circumferential direction. By providing such a rib element, an efficient means of at least partially guiding the anchor point element may be implemented, while also improving a structural rigidity of the distal anchor unit and/or the guidance element.

The second guide rail element may be a circumferential protrusion protruding from the first rib side surface in the width direction. The second guide rail element may further extend substantially along the first rib side surface along the circumferential direction. The second guide rail element may be arranged and/or provided at a predetermined height from the base plate in the radial direction. The second guide rail element may be arranged abutting the upper rib surface of the rib element. The second guide rail element may be configured to at least partially guide the anchor point element along the circumferential direction.

The guide rail may further comprise a third guide rail element. The third guide rail element may be a circumferential protrusion protruding from the second rib side surface in the width direction. The third guide rail element may further extend substantially along the second rib side surface along the circumferential direction. The third guide rail element may be arranged and/or provided at a predetermined height from the base plate in the radial direction. The third guide rail element may be arranged abutting the upper rib surface of the rib element. The third guide rail element may be configured to at least partially guide the anchor point element along the circumferential direction.

In particular, the second guide rail element and the third guide rail element may be positioned opposite one another in the width direction. Thereby, a particularly simple and efficient guide rail may be provided. However, the guide rail is not limited to such an implementation. For example, the second guide rail element and the third guide rail element may be provided at different heights along the radial direction on the first rib side surface and the second rib side surface, respectively. Thereby, for example, a correct orientation of the anchor point element on the guide rail and/or guidance element may be ensured.

One or more of the base plate, at least one guide rail, the first guide rail element, the second guide rail element, and the third guide rail element may be formed integrally. In particular, the base plate, at least one guide rail, the first guide rail element, the second guide rail element, and the third guide rail element may be formed integrally.

The guidance element may further comprise a first stopping section positioned at a first end of the guidance element along the circumferential direction, e.g., at a first end of the guide rail and/or guide groove, and a second stopping section at a second end of the guidance element, e.g., at a second end of the guidance element along the circumferential direction, e.g., at a second end of the guide rail and/or guide groove. The first stopping section and/or the second stopping section may be configured as a first and/or second stopper projection, respectively. In particular, the guidance element, the first stopping section, and/or the second stopping section may therefore be configured to prevent the anchor point element from dropping and/or running off the guidance element at the first end and/or the second end, respectively. Therefore, an unintentional dropping-off or loss of the guidance element can efficiently be prevented. The first stopping section and/or the second stopping section may be detachable from the respective first and/or second ends of the guide rail and/or the guide groove. Thereby, a facile means for mounting the anchor point element on and/or in the guidance element may be enabled. In some examples, one of the first stopping section and the second stopping section may be integrally formed with the guide rail and/or the guide groove and/or the base plate, while the other one of the first stopping section and/or the second stopping section may be detachable from the respective first and/or second ends of the guide rail and/or the guide groove. In other examples, both the first stopping section and the second stopping section may be detachable. Furthermore, the first stopping section and/or the second stopping section may be configured to at least partially guide at least one adjustment force transfer element between an adjustment actuator and the anchor point element (as described further below).

The guidance element may be configured to restrict a movement of the anchor point element along and/or parallel to an axial direction and/or a radial direction of the limb of the user. In particular, the guidance element may be configured to restrict a movement of the anchor point element along and/or parallel to the limb axis. Thereby an unintentional movement of the anchor point element along the limb may be prevented, thereby further increasing the efficiency of the assistance provided. The radial direction may further be a direction substantially perpendicular to the axial direction and/or to the limb axis. The guidance element may be further configured to restrict a movement of the anchor point element inward and/or outward along and/or parallel to the radial direction of the limb of the user. Specifically, by restricting the movement of the anchor point element inward along the radial direction an unintentional pressure of the anchor point element onto the limb may be prevented and/or reduced, which may result in an improved comfort for the wearer and a decreased risk of injury. Similarly, by restricting the movement of the anchor point element outward along the radial direction an unintentional lift-off of the anchor point element from the limb may be prevented, thereby increasing a reliability and efficiency of the assistance provided, an improved comfort for the user, and a decreased risk of injury.

The motion prediction unit may comprise at least one sensor unit. Alternatively, the motion prediction unit may be configured to receive measurement data, e.g., joint motion data of the user motion, from the at least one sensor unit. The at least one sensor unit may be configured to measure and/or determine joint motion data of the user motion. The joint motion data may comprise at least one of a current motion direction of the user motion and/or a current speed of the user motion and/or a current joint angle of the joint and/or a current joint angular velocity of the joint. The joint motion data may further comprise at least one of a current rotation orientation of the limb around the limb axis and/or a current angular velocity of the rotation orientation of the limb around the limb axis.

The joint motion data may further comprise at least one of a current elevation angle of the limb, a longitude angle of the limb, and an elbow flexion angle of the elbow joint. The elevation angle may be an angle formed by an absolute z-axis, such as a z-axis of an absolute coordinate system of the user (e.g., a world frame), and a z-axis of a coordinate system of a limb solidal frame (e.g., a local frame of the limb), such as the limb axis. A circumference may be defined centred around an origin of the limb solidal frame. A highest point of the circumference may be defined as a point on the circumference having a highest z-coordinate in the world frame. The longitude angle may be defined as an angle formed between a first line, running through the origin of the limb solidal frame and the highest point, and a y-axis of the limb solidal frame. Exemplarily, the y-axis of the limb solidal frame may be given by the position of a sensor (discussed below) placed on the limb (e.g. a user forearm), wherein the sensor has its own orientation axes xyz and its y-axis may be defined as the y-axis of the limb solidal frame. The elbow flexion angle may be defined as an angle between a limb axis of the upper arm and a limb axis of the lower arm.

In particular, the joint motion data may be indicative of a current motion state of the user motion and/or of a future motion state of the user motion. The motion prediction unit may be configured to record joint motion data as a function of time based on the measured and/or determined joint motion data.

The at least one sensor unit may further comprise at least one position sensor. The at least one position sensor may be configured to determine and/or measure a current position of the anchor point element along the circumferential direction. In particular, the determined and/or measured current position of the anchor point element may be provided to the anchor point element adjustment unit, wherein the anchor point element adjustment unit is configured to control the circumferential position of the anchor point element around the limb based on the at least partially predicted user motion of the joint and the determined and/or measured current position of the anchor point element.

For example, the at least one position sensor may comprise a first limit switch provided in the first stopping section and a second limit switch in the second stopping section. Thereby, overshoot and/or undershoot of the anchor point element around the limb can be efficiently prevented.

In particular, the motion prediction unit may be configured to at least partially predict the user motion of the joint based on the measured and/or determined and/or recorded joint motion data. For example, the motion prediction unit may be configured to at least partially predict the user motion based on the recorded joint motion data as a function of time, e.g., by extrapolation.

The motion prediction unit may be configured to at least partially predict the user motion of the joint in and/or along and/or parallel to the sagittal plane of the user. Alternatively, or additionally, the movement prediction unit may be configured to at least partially predict the user motion of the joint in and/or along and/or parallel to the frontal plane of the user.

The at least one sensor unit may comprise at least one Inertial Measurement Unit (IMU) sensor. For example, the sensor unit may comprise a first IMU sensor and a second IMU sensor. The first IMU sensor may be mountable on an upper arm of the user. The second IMU sensor may be mountable on a lower arm of the user. For instance, the second IMU sensor may be mounted on a lower part of an arm harness element. Alternatively, the second IMU sensor could be placed on a strap or a bracelet. An exemplary IMU sensor may be a BNO055 sensor.

The proximal anchor unit may comprise an anchor base mountable on the user. The anchor base may be directly mountable on the user or may be indirectly mountable on the user. For example, the anchor base may be mountable on a shoulder harness element, wherein the shoulder harness element may be mountable at least partially on a shoulder of the user.

The proximal anchor unit may further comprise a force transfer element guiding device configured to at least partially guide the force transfer element towards the anchor point element. In particular, the force transfer element guiding device may comprise a guiding cavity extending at least partially through the force transfer element guiding device. The guiding cavity may comprise a first opening on a first side of the force transfer element guiding device and a second opening on a second side of the force transfer element guiding device, wherein the guiding cavity extends from the first opening to the second opening through the force transfer element guiding device. The guiding cavity may be configured to at least partially accommodate and/or guide the force transfer element.

The proximal anchor unit may further comprise a rotational bearing provided between the anchor base and the force transfer element guiding device. The rotational bearing may be configured to allow the force transfer element guiding device to substantially freely rotate around a first rotation axis relative to the anchor base in response to a change in the circumferential position of the anchor point element around the limb.

In particular, it is therefore possible for the force transfer element guiding device to orientate itself according to the motion of the anchor point element along the circumferential direction. In other words, it may therefore be possible for the force transfer element guiding device to orientate itself according to a current position of the anchor point element along the circumferential direction, preferentially independent of a rotation state of the joint of the user, e.g., the shoulder joint. Thereby, a particularly efficient assistance may be provided to the user motion.

The rotational bearing may be a shaft configured to connect the anchor base and the force transfer element guiding device, wherein the shaft is configured to substantially freely rotate with respect to at least one of the anchor base and the force transfer element guiding device. However, the proximal anchor unit is not restricted to such a rotational bearing, and other types of bearings may equally be implemented, such as other types of plain bearings and/or ball bearings. At least one lubricating element and/or lubricating material may be positioned between the rotational bearing and at least one of the anchor base and the force transfer element guiding device.

The anchor base may comprise at least a first base element and a second base element. The first base element may be configured to be mountable on the user. The second base element may be fixedly or releasably connectable to the first base element. For example, the second base element may be releasably connectable to the first base element by tightening one or more fixation screws. As another example, the second base element may be fixedly connectable to the first base element by gluing the second base element to the first base element. It is understood, however, that the anchor base is not limited to such examples, and other means of connecting the first and second base elements may be implemented. Exemplarily, the second base element may be shaped so as to fit an additional strap to be mounted on the user for wearing of the orthosis (in addition the harness). The additional strap may further secure the orthosis to the user and prevent it from sliding (e.g. sliding off the shoulder of the user).

Furthermore, the first base element and the second base element may define therebetween a base cavity. The base cavity may be configured to at least partially receive and/or accommodate the rotational bearing. In particular, the base cavity may be configured to allow rotation, preferably substantially free rotation, of the rotational bearing relative to the anchor base. Alternatively, the rotational bearing may be fixedly connectable to the anchor base, such as fixedly connectable to the base cavity. For example, a rotational bearing fixation element, such as a screw or bolt, may be provided to fixedly connect the rotational bearing to the anchor base and/or to the base cavity. Furthermore, the base cavity may be configured to prevent an axial displacement of the received and/or accommodated rotational bearing along the first rotation axis.

Alternatively, the anchor base may be integrally formed with the rotational bearing. In such a case, the rotational bearing may be configured to allow rotation, preferably substantially free rotation, of the force transfer element guiding device relative to the rotational bearing. In such an embodiment, the number of individual parts may be reduced and a setup and composition of the anchor base may be simplified.

The force transfer element guiding device may comprise at least a first guiding element and a second guiding element. The first guiding element and the second guiding element may be configured to be connectable, preferably fixedly or releasably connectable, to one another. For example, the second guiding element may be releasably connectable to the first guiding element by tightening one or more fixation screws. As another example, the second guiding element may be fixedly connectable to the first guiding element by gluing the second guiding element to the first guiding element. It is understood, however, that the force transfer element guiding device is not limited to such examples, and other means of connecting the first and second guiding elements may be implemented.

The first guiding element and the second guiding element may define therebetween the guiding cavity.

Furthermore, the first guiding element and the second guiding element may define therebetween a guiding element base cavity. The guiding element base cavity may be configured to at least partially receive and/or accommodate the rotational bearing. In particular, the guiding element base cavity may be configured to allow rotation, preferably substantially free rotation, of the rotational bearing relative to the force transfer element guiding device. Alternatively, the rotational bearing may be fixedly connectable to the force transfer element guiding device, such as fixedly connectable to the guiding element base cavity. For example, a rotational bearing fixation element, such as a screw or bolt, may be provided to fixedly connect the rotational bearing to the force transfer element guiding device and/or to the guiding element base cavity.

Alternatively, the force transfer element guiding device may be integrally formed with the rotational bearing. In such a case, the rotational bearing may be configured to allow rotation, preferably substantially free rotation, of the anchor base relative to the rotational bearing. In such an embodiment, the number of individual parts may be reduced and a setup and composition of the force transfer element guiding device may be simplified.

The user-wearable orthosis may further comprise a pulling actuator unit configured to exert the pulling force. In particular, the pulling actuator unit may be and/or comprise a pulling actuator configured to exert the pulling force. The pulling actuator may be any kind of actuator capable of exerting, directly or indirectly, the pulling force on the at least one force transfer element. In particular, the pulling actuator may be configured to exert the pulling force on the force transfer element to assist the user motion. The pulling actuator may be a linear actuator or a rotary actuator. An exemplary pulling actuator may be an AK60-6 rotary actuator.

The pulling actuator may comprise a spool, wherein the force transfer element is at least partially wound around the spool. The pulling actuator may be configured to controllably rotate the spool around its spool axis in a first spool rotation direction to wind up the force transfer element in order to exert the pulling force on the force transfer element. The pulling actuator may be further configured to controllably rotate the spool around its spool axis in a second spool rotation direction to unwind the force transfer element from the spool, thereby providing slack in the force transfer element. In particular, the pulling actuator may be a backdriveable actuator. Alternatively, or additionally, the pulling actuator may be further configured to be switchable into a free rotation mode to allow the spool to freely rotate around its spool axis in at least the second spool rotation direction, thereby allowing the force transfer element to be unwound, e.g., as a result of user motion. The pulling actuator may be a non-backdriveable actuator.

The pulling actuator may be mountable on at least one harness element. For example, the pulling actuator may be mountable on and/or (preferably releasably) fixable to a torso harness element.

The user-wearable orthosis may further comprise the force transfer element, preferentially one force transfer element. The force transfer element may be, for example, a tendon, a cable, a strip, and/or a ribbon. The force transfer element may be guided within a Bowden sheath at least partially between the force transfer element guiding device and the pulling actuator. The user-wearable orthosis and/or the force transfer element may further comprise the Bowden sheath. In particular, the Bowden sheath may be configured to be connectable to the pulling actuator and the force transfer element guiding device.

The proximal anchor unit may further comprise a Bowden sheath abutment device configured to be connectable to one end of the Bowden sheath. The one end of the Bowden sheath may freely abut the Bowden sheath abutment device. Alternatively, the one end of the Bowden sheath may be fixedly or releasably mountable to the Bowden sheath abutment device. The Bowden sheath and the Bowden sheath abutment device may be connected by means of any cable gland. Exemplarily, the Bowden sheath abutment device may be 3D printed and may be secured to the Bowden sheath with a heat insert and a screw.

The Bowden sheath abutment device may be connectable to the force transfer element guiding device. In particular, the Bowden sheath abutment device and the force transfer element guiding device may be configured to be substantially freely rotatable relative to one another around a second rotation axis in response to the change in the circumferential position of the anchor point element around the limb. In particular, by providing a proximal anchor unit having both the rotational bearing and the Bowden sheath abutment device a disengagement of a rotation of the proximal anchor unit and/or of the user-wearable orthosis and a rotation of the joint, e.g., the shoulder joint, and/or of the limb, e.g., around the limb axis, may be achievable.

Furthermore, the first guiding element and the second guiding element may define therebetween an abutment cavity. The abutment cavity may be configured to at least partially receive and/or accommodate the Bowden sheath abutment device. In particular, the abutment cavity may be configured to allow rotation, preferably substantially free rotation, of the Bowden sheath abutment device relative to the force transfer element guiding device. In particular, the first opening may open into the abutment cavity.

The pulling actuator may further comprise a pulling actuator abutment section configured to be connectable to a second end of the Bowden sheath. The second end of the Bowden sheath may freely abut the pulling actuator abutment section. Alternatively, the second end of the Bowden sheath may be fixedly or releasably mountable to the pulling actuator abutment section. The pulling actuator abutment section may be connectable to the pulling actuator or form part of the pulling actuator.

The first rotation axis may be substantially identical to the second rotation axis. In particular, such a configuration of the proximal anchor unit may allow for a very efficient and facile operation of the user-wearable orthosis.

The anchor point element adjustment unit may further comprise an adjustment actuator connectable, via at least one adjustment force transfer element, to the anchor point element. The anchor point element adjustment unit may comprise the at least one adjustment force transfer element. The at least one adjustment force transfer element may be configured to have a force, such as an adjustment pulling force, exerted thereon. The adjustment actuator and the pulling actuator may be provided and/or housed in a common actuation unit. The pulling actuator may be configured to generate a power and/or a torque that is larger than a power and/or torque, respectively, generatable by the adjustment actuator. Furthermore, the adjustment actuator may be smaller dimensioned than the pulling actuator. In particular, it may therefore be possible to provide the user-wearable orthosis with reduced power and/or size requirements.

Accordingly, in some examples, the orthosis may comprise a pulling actuator and an adjustment actuator. It is particularly advantageous that the control of the user-wearable orthosis is simplified because the actuators are not working in parallel on the same joint but rather one for assistance (the pulling actuator) and the other for reconfiguration (the adjustment actuator).

Exemplarily, the two actuators may be implemented using the same type of device (e.g. they may both be AK60-6). In this way, the actuators may rely on the same CAN-bus communication, making the electronics implementation more compact. However, they may have different limits in terms of torque output, wherein the adjustment actuator may require less torque with respect to the pulling actuator.

Each adjustment force transfer element may be, for example, a tendon, a cable, a strip, and/or a ribbon. Each adjustment force transfer element may be guided within at least one, for example two, adjustment Bowden sheath(s) at least partially between the anchor point element and the adjustment actuator. The user-wearable orthosis and/or the adjustment force transfer element may further comprise the at least one adjustment Bowden sheath. In particular, the at least one adjustment Bowden sheath may be configured to be connectable to the adjustment actuator and the distal anchor unit and/or the anchor point element. In particular, the first stopping section and/or the second stopping section may each be configured to be connectable to one end of at least one adjustment Bowden sheath.

The adjustment actuator may be configured to exert a force, for example the adjustment pulling force, on the at least one adjustment force transfer element to control the circumferential position of the anchor point element. In particular, the adjustment actuator may be configured to exert the adjustment pulling force on the at least one adjustment force transfer element to change a current position of the anchor point element along the circumferential direction to a desired position, e.g., the localised position, of the anchor point element along the circumferential direction. In particular, the current position may be different from the desired position. For example, the adjustment actuator may comprise a spool for each adjustment force transfer element, wherein the adjustment actuator may be configured to rotate the spool of a respective adjustment force transfer element in order to exert the adjustment pulling force on said respective adjustment force transfer element.

In particular, the at least one adjustment force transfer element may be a circular tendon having a first end and a second end. Both the first end and the second end of the tendon may be fixed and/or fixable to the anchor point element. Alternatively, both the first end and the second end of the tendon may be fixed and/or fixable to the adjustment actuator, e.g., a spool of the adjustment actuator.

In particular, the first end of the circular tendon may be fixed to the anchor point element, wherein the circular tendon may extend from the first end at the anchor point element at least partially along the distal anchor unit, such as at least partially along the guidance element to the first stopping section. The distal anchor unit and/or the first stopping section may be configured to guide the circular tendon towards the adjustment actuator. The circular tendon may be wound around the spool of the adjustment actuator and extend from the adjustment actuator back towards the distal anchor unit and/or towards the second stopping section. The circular tendon may be guided by the distal anchor unit and/or the second stopping section and extend along the distal anchor unit and/or the guidance element to the anchor point element. The second end of the circular tendon may be fixed to the anchor point element. Please note that the above is described for the first and second ends being fixed to the anchor point element. However, the user-wearable orthosis is not limited to such an implementation. For example, the first and second ends of the circular tendon may alternatively be fixed to the adjustment actuator, such as for example the spool of the adjustment actuator.

Please note that, while the circular tendon has been described as a singular tendon above, the user-wearable orthosis is not limited to such an implementation. In particular, two or more adjustment force transfer element may be provided. For example, a first tendon may connect the adjustment actuator to the anchor point element such that exerting an adjustment pulling force on the first tendon may move the anchor point element in one direction along the circumferential direction, and a second tendon may connect the adjustment actuator to the anchor point element such that exerting an adjustment pulling force on the second tendon may move the anchor point element in the other, opposite direction along the circumferential direction. The adjustment actuator may in particular be configured to exert the adjustment pulling force on one of the first tendon and the second tendon, while releasing and/or unwinding the other one of the first tendon and the second tendon, and vice versa.

Alternatively, the at least one adjustment force transfer element may be a closed loop tendon. The closed loop tendon may extend from the anchor point element, either directly or indirectly, to the adjustment actuator and from the adjustment actuator, either directly or indirectly, back to the anchor point element.

In another embodiment, the anchor point element adjustment unit may comprise an adjustment force transfer element connected, at a first end thereof, to the adjustment actuator, and at a second end thereof, to the anchor point element. The adjustment actuator may be configured to exert the adjustment pulling force on the adjustment force transfer element to cause the anchor point element to move in one direction along the circumferential direction. The distal anchor unit may further comprise a restoring element, such as a spring element. The restoring element may be configured to be connectable at one end to the distal anchor unit, e.g., to the first stopping section or the second stopping section. The restoring element may be further configured to be connectable at a second end to the anchor point element. The restoring element may be further configured to exert a restoring pulling force on the anchor point element to move the anchor point element to a neutral position along the circumferential direction. The neutral position may be freely chosen, and the restoring element may be accordingly configured. For example, the neutral position may be located at one of the ends of the circumferential range. Therefore, the anchor point element may remain at the neutral position or be restored to the neutral position when no adjustment pulling force is exerted thereon. In particular, a design and operation of the user-wearable orthosis may thereby be simplified.

While the above has been described in relation to only one restoring element, the user-wearable orthosis is not limited to such an implementation. For example, two restoring elements may be provided in order to allow setting of the neutral position to any location along the circumferential direction. In particular, the two restoring elements may comprise a first restoring element and a second restoring element, wherein a first restoring pulling force exertable by the first restoring element may be directed along the circumferential direction in a first direction, wherein a second restoring pulling force exertable by the second restoring element may be directed along the circumferential direction in a second direction opposite to the first direction.

The adjustment actuator may be configured to be worn by the user on a torso or back of the user, preferentially on a central portion of the torso or back of the user. For example, the adjustment actuator may be mountable on a torso harness element. By wearing the adjustment actuator on a back of a user, a weight of the user-wearable orthosis may be more efficiently distributed, particularly in light of a weight of the adjustment actuator itself. Furthermore, a comfort of wear may thereby be increased.

The adjustment actuator may be any kind of actuator capable of exerting, directly or indirectly, the adjustment pulling force on the (at least one) adjustment force transfer element. The adjustment actuator may be a linear actuator or a rotary actuator. An exemplary adjustment actuator may be an AK60-6 rotary actuator.

The adjustment actuator may be provided in the distal anchor unit. Specifically, the adjustment actuator may therefore be provided close to the anchor point element, which may simplify the design and setup of the user-wearable orthosis. Furthermore, a reliability of the user-wearable orthosis may be significantly increased, as a chance of the adjustment force transfer element suffering damage or catching on external objects can be reduced. Furthermore, by providing the adjustment actuator in the distal anchor unit, a weight of the adjustment actuator may be at least partially worn and/or borne and/or supported by the pulling actuator.

The adjustment actuator may, for example, be provided in the guidance element, such as in the guide rail. Alternatively, the adjustment actuator may be provided in the first stopping section and/or in the second stopping section.

The anchor point element adjustment unit may further comprise an element adjustment actuator configured to control the circumferential position of the anchor point element. In particular, the element adjustment actuator may be provided in the anchor point element. Thereby, a particularly compact and efficient design of the user-orthosis may be provided. The element adjustment actuator may be an electrical motor configured to propel and/or move the anchor point element along the circumferential direction, such as for example along the guidance element. The element adjustment actuator may be supplied with power via, for example, the distal anchor unit and/or the guidance element.

The anchor point element may be configured to comprise a force transfer element connection point, wherein the force transfer element may be fixedly or releasably connectable to the force transfer element connection point. The anchor point element may further comprise a movement base element. The movement base element may, for example, be configured as a cart and/or sled. The movement base element may comprise at least one, preferably four, wheel elements fixedly connected to the movement base element. In particular, each wheel element may comprise a wheel axis and a wheel, wherein the wheel axis is fixedly connected to the movement base element, and wherein the wheel is rotatable around the wheel axis. The at least one wheel element may be configured to be engageable with the guidance element, wherein each wheel of the at least one wheel element may be in contact with the guidance element. Furthermore, each wheel may be configured to roll along the guidance element during movement of the anchor point element along the guidance element and/or the circumferential direction. At least one wheel element may be provided to engage the first rib side surface and at least one wheel element may be provided to engage the second rib side surface.

Please note that while a preferred example of the number of wheel elements is given as four above, the user-wearable orthosis is not restricted to such a number. In particular, one, two, three, or five and more wheel elements may be provided.

In a particular exemplary embodiment, the joint may be a shoulder joint of the user, the limb may be an arm and/or upper arm of the user, and the user motion of the joint may be a lifting of the arm of the user. In an alternative, exemplary embodiment, the joint may be a hip joint of the user, the limb may be a leg and/or a thigh of the user, and the user motion of the joint may be a lifting of the leg of the user.

The user-wearable orthosis may further comprise at least one control unit. The at least one control unit may in particular comprise at least the motion prediction unit. The at least one control unit may be configured to receive data inputs from one or more parts of the user-wearable orthosis. For example, the at least one control unit may be configured to receive input from the at least one sensor unit, e.g., from the first and second IMUs, for instance via Bluetooth communication. An exemplary control unit may be an Arduino MKR1010WiFi.

The at least one control unit may be a single central control unit. The single central control unit may be configured to receive data input from, provide data output to, and/or control operation of multiple parts of the user-wearable orthosis, such as for example the motion prediction unit and/or the at least one sensor unit and/or the pulling actuator and/or the adjustment actuator.

However, the user-wearable orthosis is not limited to such an implementation, wherein, for example, the at least one control unit may alternatively comprise a plurality of control units. For example, at least one control unit may comprise a pulling actuator control unit configured to control the operation of the pulling actuator and/or an adjustment actuator control unit configured to control the operation of the adjustment actuator. The anchor point element adjustment unit may comprise at least the adjustment actuator control unit.

The motion prediction unit may be configured to receive the joint motion data from the at least one sensor unit. For example, the motion prediction unit may be configured to receive at least the longitude angle $\beta$.

Furthermore, the inventors realised during the conception of the present invention, that for small elevation angles $\alpha$, the longitude angle $\beta$ may change abruptly. Therefore, the motion prediction unit may be further configured to also receive the elevation angle $\alpha$, wherein the motion prediction unit may be configured to filter the longitude angle $\beta$ based on the elevation angle $\alpha$, and output the filtered longitude angle $\beta_{filt}$ to the anchor point element adjustment unit. Thereby, a smooth variation of the longitude angle $\beta$ may be achieved even for small elevation angles $\alpha$. Alternatively, the motion prediction unit may also be configured to output the (unfiltered) longitude angle $\beta$ to the anchor point element adjustment unit. Thereby, a simple and efficient motion prediction unit may be enabled.

In particular, the motion prediction unit may be configured to receive the elevation angle $\alpha$ and the longitude angle $\beta$, and may be further configured to apply an exponential moving average (EMA) adaptive filter, as a function of the elevation angle $\alpha$, to the longitude angle $\beta$. The EMA adaptive filter may be a low-pass, discrete, Infinite-Impulse Response (IIR) filter. Such a filter may behave similar to a discrete RC low-pass filter.

A difference equation of an EMA filter may be a weighted combination of the newest input data with the previous output. The respective weights may be configured in such a way that their sum equals 1. An equation of the EMA filter may be given by (equation A.1):

$$y[n] = (1 - \lambda)x[n] + \lambda y[n - 1]$$

Here, $y[n]$ is the output at the current time step $n$, $x[n]$ is the input at the current time step $n$, $y[n - 1]$ is the output at the previous time step $(n - 1)$, and $\lambda$ is a parameter corresponding to a strength and/or aggressiveness of the filter. $\lambda$ may in particular be a constant in the interval of [0,1]. For $\lambda = 0$, the current output is identical to the current input, and so no filtering is undertaken. For $\lambda = 1$, the current output is identical to the previous output, and so the filtering is maximal. In other words, a cut-off frequency of the system increases with increasing value of $\lambda$.

In particular, equation A.1 may be rewritten as (equation A.2):

$$y[n] = (1 - \lambda) \sum_{k=0}^{n} \lambda^k x[n - k]$$

An estimation of a time constant $t_c$ of the EMA filter may be approximated for systems having a fixed time step $T$ as (equation A.3):

$$t_c \approx -\frac{T}{\ln(\lambda)}$$

This approximation may hold for cut-off frequencies far from the Nyquist rate. The approximation may be useful for evaluating an approximation of the delay that the EMA filter introduces. Typically, it may take around 5 time constants for a signal to reach approximately 99% of a final value in a step response.

In the EMA adaptive filter applied by the motion prediction unit, the value of $\lambda$ may be implemented as a variable function $\lambda(\alpha)$ of the elevation angle $\alpha$. In particular, a value of $\lambda$ may be chosen between a maximal value $\lambda_{max}$ and a minimal value $\lambda_{min}$. For example, for small elevation angles $\alpha$, a value of $\lambda$ may be chosen close to 1 in order to make the system less responsive to abrupt changes in the longitude angle $\beta$. Conversely, for large elevation angles $\alpha$, a value of $\lambda$ may be chosen close to 0 in order to make the system more responsive to changes in the longitude angle $\beta$.

In particular, the variable function $\lambda(\alpha)$ may be chosen as a convex combination between its maximal and minimal values, and may be given as (equation A.4):

$$\lambda(\alpha) = \big(1 - f(\alpha)\big)\lambda_{max} + f(\alpha)\lambda_{min}$$

In particular, $f(\alpha)$ may be a custom sigmoid function allowing for a smooth transition between the maximal and minimal values, and may be given as (equation A.5):

$$f(\alpha) = \frac{1}{1 + e^{-g(\alpha)}}$$

The function $g(\alpha)$ may be a linear function mapping the elevation angle $\alpha$ to an x-coordinate of the sigmoid function $f(\alpha)$. In particular, the function $g(\alpha)$ may be determined based on the specific requirements of the user and/or the user-wearable orthosis. For example, it may be desired that the filter parameter $\lambda(\alpha)$ assumes values close to $\lambda_{max}$ for elevation angles $\alpha$ lower than 20°. Furthermore, as an example, it may be desired that the filter parameter $\lambda(\alpha)$ assumes values close to $\lambda_{min}$ for elevation angles $\alpha$ greater than 40°. Furthermore, it may be assumed that the most variation in the sigmoid function $f(\alpha)$ may occur in the x-coordinate range [-2.5, 2.5], a line passing through points (20, -2.5) and (40, 2.5) may be chose (as equations A.6):

$$\frac{\alpha - 20}{40 - 20} = \frac{x + 2.5}{2.5 + 2.5}$$

$$x = \frac{\alpha - 30}{4}$$

By changing the denominator of equation A.6 above, a slope of the sigmoid function $f(\alpha)$ may be adjusted: the smaller the denominator, the higher the slope.

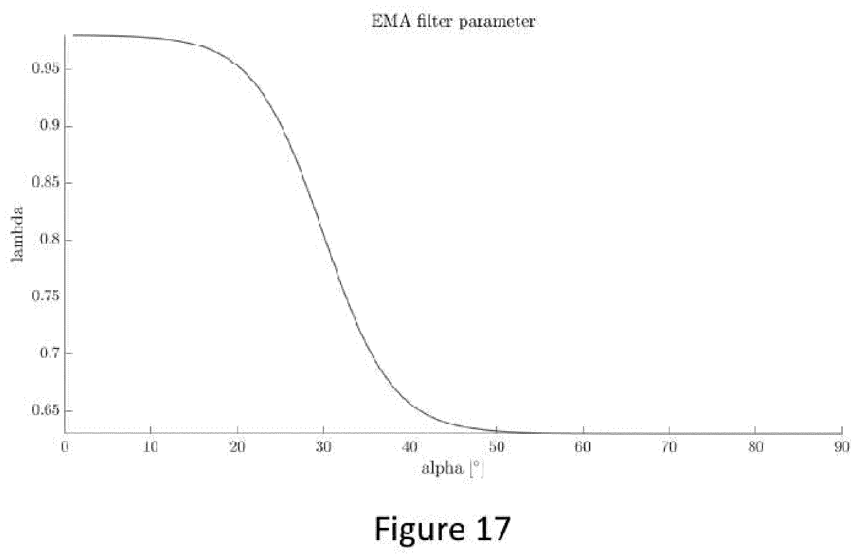

For a graphical representation of $\lambda(\alpha)$ of the above example, reference is made to Figure 17.

Therefore, the motion prediction unit may be configured to apply an EMA adaptive filter, as a function of the elevation angle $\alpha$, to the longitude angle $\beta$ in order to obtain a filtered longitude angle $\beta_{filt}$. The filtered longitude angle $\beta_{filt}$ may be output to the anchor point element adjustment unit.

The anchor point element adjustment unit may be configured to receive both the filtered longitude angle $\beta_{filt}$ and a feedback motor position $\beta_{fb}$. The feedback motor position $\beta_{fb}$ may correspond to a current motor position of the adjustment actuator, wherein the adjustment actuator may be configured to output said feedback motor position $\beta_{fb}$ to the anchor point element adjustment unit.

It is noted that, even if proportional, these two quantities (the filtered longitude angle $\beta_{filt}$ and the feedback motor position $\beta_{fb}$) are different. In order to make them coherent, the anchor point element adjustment unit may be configured to convert one of the two quantities. The conversions may comprise a proportion, which may be determined by measuring the two respective quantities at two different and pre-defined positions around the limb of the user.

The anchor point element adjustment unit may then be configured to determine a difference $\beta_{error}$ between the filtered longitude angle $\beta_{filt}$ and the feedback motor position $\beta_{fb}$, wherein said difference $\beta_{error}$ may be output to the adjustment PID position controller in order to control the position of the anchor point element. Parameters of the adjustment PID position controller may be tuned using the Ziegler-Nichols method. The adjustment PID position controller may further comprise an internal PID velocity controller to handle a low-level control of the adjustment PID position controller.

The pulling actuator control unit may be an indirect force controller that maps a desired torque profile to a corresponding velocity profile of the pulling actuator.

Specifically, the pulling actuator control unit may comprise a gravitational torque evaluator (GTE) module, an applied torque estimator (ATE) module, and an admittance controller module. Based on data input, specifically, the joint motion data, from the at least one sensor unit, the GTE module may be configured to evaluate a gravitational torque that should be compensated by the user-wearable orthosis. The ATE module may be configured to estimate a torque applied by the pulling actuator. The pulling actuator control unit may be configured to determine an error and/or difference between the evaluated gravitational torque and the estimated applied torque. Finally, the admittance controller may be configured to translate the determined error and/or difference into a velocity reference, which may be passed to a PID velocity controller of the pulling actuator. The PID velocity controller may be configured to handle the low-level control of the pulling actuator.

The GTE module may be configured to receive the joint motion data from the at least one sensor unit. For example, the GTE module may be configured to receive the elevation angle $\alpha$, the longitude angle $\beta$, and the elbow flexion angle $\theta_e$.

The gravitational compensation torque $\tau_g$ acting on the virtual joints corresponding to the elevation angle $\alpha$ may be given by (equation B.1):

$$\tau_g = -\frac{d}{dt}\frac{\partial U}{\partial \dot{\alpha}} + \frac{\partial U}{\partial \alpha}$$

The arm of the user may be treated as a kinematic chain consisting of rigid bodies interconnected by joints. The hand may be assumed to be rigidly attached to the lower arm (forearm), disregarding the wrist joint. A general formulation for the potential energy of a kinematic chain with m links may be given by (equation B.2):

$$U = \sum_{i=1}^{m} m_i g z_i$$

where $m_i$ may be the mass of the i-th link, $z_i$ may be the z-coordinate of the centre of mass of the i-th link, and $g$ may be the gravitational acceleration.

Since, as discussed above, the wrist joint may be disregarded, the arm may be simplified as a 2-link kinematic chain having a first link corresponding to the upper arm and a second link corresponding to the lower arm and hand.

Taking the world frame as a reference, a z-coordinate $z_u$ of the centre of mass of the first link may be given by (equation B.3):

$$z_u = {}^0_3T \begin{bmatrix} 0 \\ 0 \\ -l_{Gu} \\ 1 \end{bmatrix} = \begin{bmatrix} & & & 0 \\ & {}^0_3R & & 0 \\ & & & 0 \\ 0 & 0 & 0 & 1 \end{bmatrix} \begin{bmatrix} 0 \\ 0 \\ -l_{Gu} \\ 1 \end{bmatrix} = -l_{Gu}c(\theta_2)c(\theta_1)$$

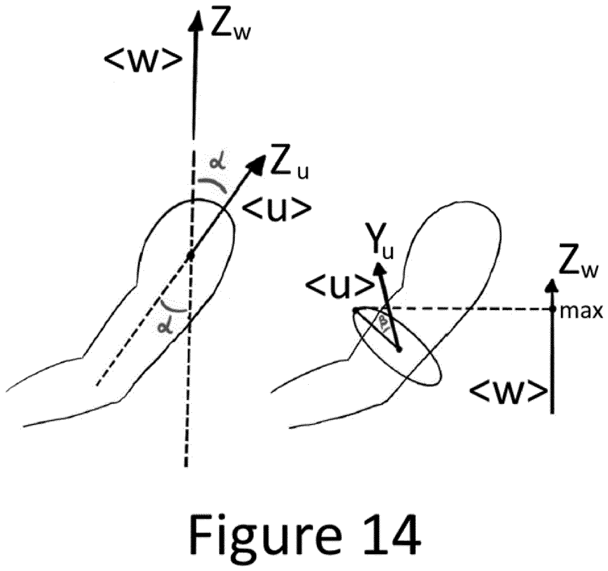

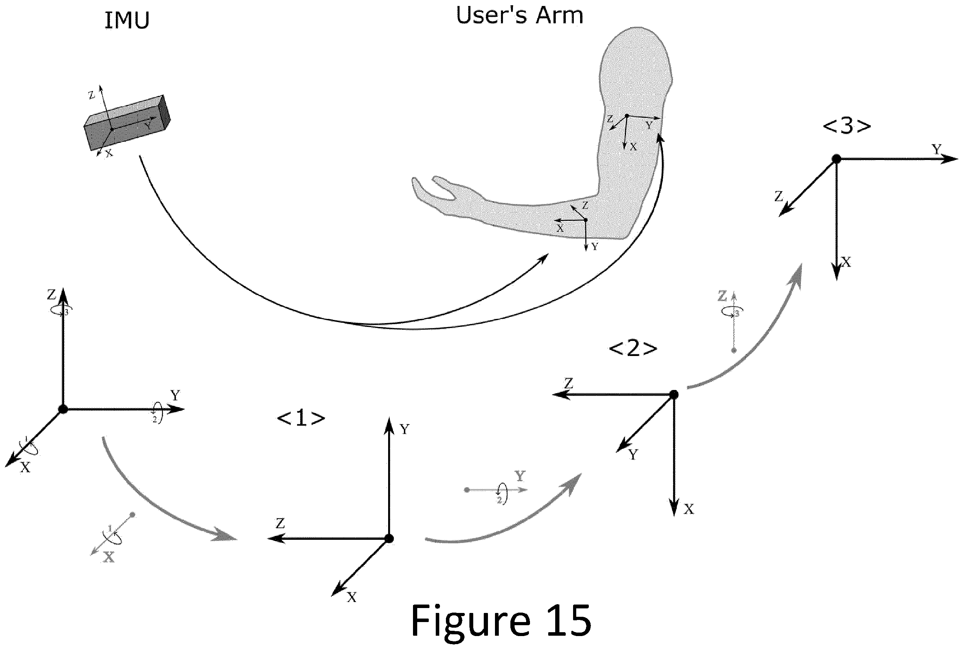

Reference is made to Figures 14 and 15, and their corresponding figure descriptions below for an overview of the used notation and parameters.

Furthermore, the parameter $l_{Gu}$ may correspond to a distance between the centre of mass of the first link and the origin of the last frame of the XYZ Euler angle parametrisation rotation sequence. The matrix ${}^0_3T$ may be a matrix identifying a transformation between the last frame of the XYZ Euler angle parametrisation rotation sequence and the world frame.

Taking the world frame as a reference, a z-coordinate $z_l$ of the centre of mass of the second link may be given by (equation B.4):

$$z_l = {}^0_3T\,{}^3_4T \begin{bmatrix} 0 \\ 0 \\ -l_{Gl} \\ 1 \end{bmatrix} = \begin{bmatrix} & & & 0 \\ & {}^0_3R & & 0 \\ & & & 0 \\ 0 & 0 & 0 & 1 \end{bmatrix} \begin{bmatrix} 1 & 0 & 0 & 0 \\ 0 & c(\theta_e) & -s(\theta_e) & 0 \\ 0 & s(\theta_e) & c(\theta_e) & -l_u \\ 0 & 0 & 0 & 1 \end{bmatrix} \begin{bmatrix} 0 \\ 0 \\ -l_{Gl} \\ 1 \end{bmatrix}$$

$$= -l_{Gl}c(\theta_2)c(\theta_1 + \theta_e) - l_u c(\theta_2)c(\theta_1)$$

The parameter $l_u$ may correspond to a length of the first link. The parameter $l_{Gl}$ may correspond to a distance between the centre of mass of the second link and the origin of the elbow frame.

The matrix ${}^3_4T$ may be a matrix identifying a transformation between the elbow frame and last frame of the XYZ Euler angle parametrisation rotation sequence of the shoulder. In particular, a rotation part of the matrix ${}^3_4T$ may correspond to a rotation by angle $\theta_e$ around the x-axis.

Based on the above, a potential energy of the arm may be obtained by the following equation (equation B.5):

$$U = m_u g z_u + m_l g z_l$$

$$= -gc(\theta_2)\{c(\theta_1)[(m_u l_{Gu} + m_l l_u) + m_l l_{Gl} c(\theta_e)] - s(\theta_1)[m_l l_{Gl} s(\theta_e)]\}$$

Therefore, a z-coordinate of the centres of mass and, as a consequence, also the potential energy of the arm do not depend on the third Euler angle $\theta_3$ of the XYZ Euler angle parametrisation.

Furthermore, the following notation may be used (equations B.6):

$$K_{ul} = m_u l_{Gu} + m_l l_u$$

$$K_l = m_l l_{Gl}$$

Therefore, using equations B.6 and equations 15.17 (see figure description of Figure 15 below), equation B.5 may be rewritten as (equation B.7):

$$U = -g\{c(\alpha)[K_{ul} + K_l c(\theta_e)] - s(\alpha)c(\beta)[K_l s(\theta_e)]\}$$

Equation B.7 may be substituted into equation B.1, wherein it may be further assumed that the potential energy does not depend on $\dot{\alpha}$, to give the following equation for the gravitational compensation torque $\tau_g$ (equation B.8):

$$\tau_g = 0 + \frac{\partial U}{\partial \alpha} = g\{s(\alpha)[K_{ul} + K_l c(\theta_e)] + c(\alpha)c(\beta)[K_l s(\theta_e)]\}$$

The pulling actuator control unit and/or the GTE module may be further configured to also account for damping due to biological articulations and bones. Specifically, out of the three angles contained in equations B.8, only $\alpha$ and $\theta_e$ may be relevant for modelling such damping. Therefore, equation B.8 may be extended to include two additional damping terms dependent on the velocities of the respective angles $\alpha$ and $\theta_e$. Therefore, equations B.8 may be rewritten to account for the damping as follows (equation B.9):

$$\tau_g = g\{s(\alpha)[K_{ul} + K_l c(\theta_e)] + c(\alpha)c(\beta)[K_l s(\theta_e)]\} + D_1 \dot{\alpha} + D_2 \dot{\theta}_e$$

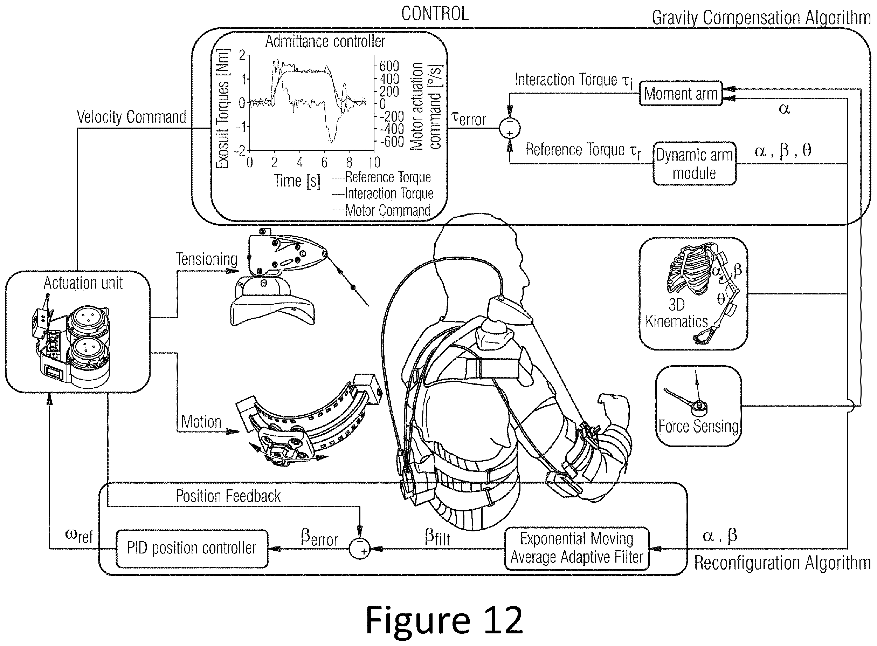

$D_1$ and $D_2$ may be damping constants and may be empirically determined. The gravitational compensation torque $\tau_g$ may further be referred to as reference torque $\tau_r$, as shown, for example in Figure 12.

The ATE module may be configured to estimate a torque applied by the pulling actuator, preferentially to the force transfer element and/or the anchor point element. Specifically, the ATE module may be configured to estimate a torque deriving from a tension in the force transfer element and that is applied at the actuated virtual joint corresponding to angle $\alpha$. For the following, reference is made to Figures 14 to 16, and their corresponding figure descriptions below.

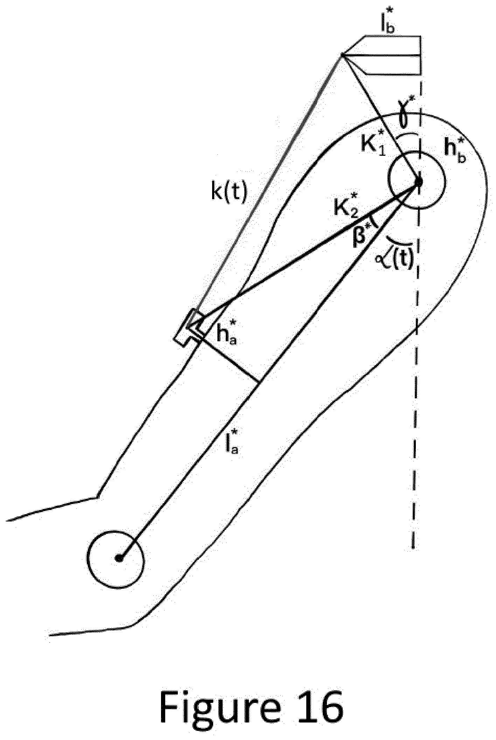

In particular, a model of the limb, e.g., the arm, of the user with the user-wearable orthosis may be derived. Such an exemplary model is shown in Figure 16, described further below. It is noted that the model may be assumed to be two-dimensional. This is possible due to the specific design of the user-wearable orthosis, as described herein.

Specifically, by providing a proximal anchor unit as described herein, it becomes possible to decouple a rotational orientation of the force transfer element guiding device from a rotational state of the Bowden sheath and a rotational state of the shoulder. Thus, the force transfer element guiding device of the proximal anchor unit is capable of rotating around the first and second rotation axis independently of the Bowden sheath and the shoulder. The force transfer element guiding device may therefore passively rotate around the first and second rotation axis according to a circumferential position of the anchor point element. Consequently, the force transfer element guiding device and the anchor point element may always be maintained on the same plane, wherein the elevation angle $\alpha$ is defined in said plane. The joint, such as the shoulder joint, may therefore be considered a simple revolute joint.

Furthermore, other assumptions may be taken into account. For example, as already discussed above, the limb and joint may be modelled as a kinematic chain, e.g., a 2-link kinematic chain. Furthermore, it may also be assumed that the user-wearable orthosis does not move and/or shift with respect to the user in the mounted state. This may, for example, be achieved by appropriately forming the user-wearable orthosis and/or providing appropriate at least one intermediate elements, as discussed previously.

Exemplarily, the assistance efficacy of the device may be maximal when the force transfer element guiding device is parallel to the ground.

In particular, it may be possible to derive a length of the force transfer element between the proximal anchor unit, e.g., the second opening, and the anchor point element, e.g., the force transfer element connection point, as a function of the elevation angle $\alpha$. This may be given by (equation B.10):

$$k(\alpha) = \sqrt{k_1^{*2} + k_2^{*2} - 2k_1^* k_2^* c\big(\pi - (\gamma^* + \beta^* + \alpha)\big)}$$

$$= \sqrt{k_1^{*2} + k_2^{*2} + 2k_1^* k_2^* c(\gamma^* + \beta^* + \alpha)}$$

Given $k(\alpha)$, a force transfer element displacement $\Delta k(\alpha)$, with respect to the length of the force transfer element when the limb is at rest ($\alpha = 0$), may be obtained by the following equation (equation B.11):

$$\Delta k(\alpha) = \sqrt{k_1^{*2} + k_2^{*2} + 2k_1^* k_2^* c(\gamma^* + \beta^* + \alpha)} - \sqrt{k_1^{*2} + k_2^{*2} + 2k_1^* k_2^* c(\gamma^* + \beta^*)}$$

A variation of the force transfer element displacement with varying elevation angle $\alpha$ may be described by the derivative of $\Delta k(\alpha)$ with respect to the elevation angle $\alpha$. Said derivative $J(\alpha)$ may be given by (equation B.12):

$$J(\alpha) = \frac{\partial \Delta k(\alpha)}{\partial \alpha} = -\frac{k_1^* k_2^* s(\gamma^* + \beta^* + \alpha)}{\sqrt{k_1^{*2} + k_2^{*2} + 2k_1^* k_2^* c(\gamma^* + \beta^* + \alpha)}}$$

Therefore, the torque estimated by the ATE module, also referred to as interaction torque $\tau_i$ (see, e.g., Figure 12), may therefore be given by (equation B.13):

$$\tau_i = J(\alpha)f$$

Here, $f$ may correspond to a tension of the force transfer element. In particular, $f$ may be determined and/or measured by a load sensor, as described further below.

The pulling actuator control unit may be further configured to determine a torque difference or torque error $\tau_{error}$ between the reference torque $\tau_r$ and the interaction torque $\tau_i$.

The torque error $\tau_{error}$ may be provided to the admittance controller module. Mechanical admittance may describe a force/velocity relation of an end-effector of a kinematic chain. Specifically, in the Laplace Domain, the relation may be given by (equation B.14):

$$\frac{\dot{x}(s)}{\tau(s)} = R(s) = B_a + \frac{D_a}{s} + M_a s$$

Here, $\tau(s)$ may correspond to a torque reference, and $B_a$, $D_a$, and $M_a$ may be design parameters.

In the admittance control module the above admittance relation may be directly applied. The admittance control module may then have a same form as a PID control, wherein $B_a$, $D_a$, and $M_a$ may be regarded as proportional, integral, and derivative gains, respectively. Thus, in the time domain (equation B.15):

$$\dot{x} = B_a \tau + D_a \int_0^t \tau(v)dv + M_a \dot{t}$$

Furthermore, the arm musculoskeletal system may be modelled as a second-order biomechanical system, wherein a frequency response of a second-order system may represent the behaviour of a low-pass filter. A cut-off frequency of such a low-pass filter may be directly related to biomechanical parameters of the second-order system. Therefore, the admittance control unit may be tuned as a function of the second-order system parameters using, for example, a Ziegler-Nichols method.

Therefore, based on the received torque error $\tau_{error}$, the admittance control module may be configured to determine and output a velocity command to the pulling actuator to exert the pulling force on the force transfer element.

The at least one control unit may further comprise a position verification module. The position verification module may be configured to obtain a current position of the anchor point element along the circumferential direction, for example from the at least one position sensor. Furthermore, the position verification module may be further configured to obtain, for example from the movement prediction unit and/or the anchor point element adjustment unit, the localised position. The position verification module may be configured to compare the localised position with the current position to determine whether the localised position matches the current position.

If the position verification module determines that the localised position matches the current position, the position verification module may be configured to enable and/or allow the pulling actuator to exert the pulling force on the force transfer element. If the position verification module determines that the localised position does not match the current position, the position verification module may be configured to prevent the pulling actuator from exerting the pulling force on the force transfer element.

In particular, the position verification module may be configured to determine that the localised position matches the current position, if the localised position substantially exactly matches the current position. The current position may substantially exactly match the current position, if an error and/or difference between the current position and the localised position is at most approx. 1% of the total circumferential range, preferably at most approx. 0.5% of the total circumferential range, further preferably at most approx. 0.1 % of the total circumferential range. Therefore, a precision of the user-wearable orthosis in the assistance provided may be significantly increased. In particular, substantially no deviation from the intended user motion is thus affected by the user-wearable orthosis, thereby improving ease of use and comfort. In addition, an acclimatisation time to the user-wearable orthosis by the user may be reduced.

Alternatively, the position verification module may be configured to determine that the localised position matches the current position, if the current position is determined to be within an acceptable range of the localised position. The acceptable range may be set as a matter of user preference. For example, the acceptable range may be at most approx. 15% of the total circumferential range, preferably at most approx. 10% of the total circumferential range, further preferably at most approx. 5% of the total circumferential range. By implementing such an acceptable range, the user-wearable orthosis may be configured to already start assisting the user even when the anchor point element hasn't fully reached the localised position. Thereby, the response time of the user-wearable orthosis can be significantly increased/improved.

The user-wearable orthosis may further comprise a power supply unit. The power supply unit may be configured to supply power to the user-wearable orthosis. For example, the power supply unit may be configured to supply power to the at least one sensor, the movement prediction unit, the at least one control unit, the pulling actuator, and/or the adjustment actuator. The power supply may be a power bank.

The user-wearable orthosis may further comprise a load sensor configured to measure the pulling force, in particular a magnitude of the pulling force, exerted by the pulling actuator and/or a tension of the force transfer element. The load sensor may be a load cell, wherein the load cell may be located and/or arranged between the force transfer element and the anchor point element, such as between the force transfer element and the force transfer element connection point. In particular, the load cell may be connected to both the force transfer element and to the anchor point element in order to measure the force exerted via the force transfer element on the anchor point element.

Preferentially, a weight of the user-wearable orthosis is at most approx. 5kg, preferably at most approx. 4kg, further preferably at most approx. 3kg, yet further preferably at most 2kg. The weight may not depend on the intended user and/or application. Specifically, it was recognised by the inventors within the scope of the present invention that the overall weight of an orthosis may significantly impact the use of said orthosis via additional stresses caused on the respective joint(s). However, by providing the user-wearable orthosis within the above weight restrictions, it is possible to significantly reduce and/or avoid said additional stresses on the respective joint(s).

A further aspect relates to a method for controlling a user-wearable orthosis for assisting a user motion of a joint of a user. The method comprises at least partially predicting the user motion of the joint, and controlling a circumferential position of an anchor point element of a distal anchor unit around a limb attached to the joint based on the at least partially predicted user motion of the joint. The distal anchor unit is mounted on the limb, wherein a proximal anchor unit mounted on or proximal to the joint is connectable to the anchor point element via a force transfer element, wherein the user-wearable orthosis is configured to exert a pulling force on the force transfer element to assist the user motion. The anchor point element is moveable at least partially circumferentially around the limb of the user.

Furthermore, features and explanations described herein for the user-wearable orthosis also apply, correspondingly, to the method for controlling a user-wearable orthosis. Consequently, the method for controlling the user-wearable orthosis may comprise, correspondingly, any combination of features, as described herein or shown in the Figures for the user-wearable orthosis.

A further aspect relates to a non-transitory computer readable storage medium containing instructions that, when executed by at least one computing device, cause the computing device to perform the method for controlling the user-wearable orthosis, as described herein.

Furthermore, features and explanations described herein for the user-wearable orthosis and the method for controlling the user-wearable orthosis also apply, correspondingly, to the non-transitory computer readable storage medium. Consequently, the non-transitory computer readable storage medium may comprise, correspondingly, any combination of features, as described herein or shown in the Figures for the user-wearable orthosis and the method of controlling the user-wearable orthosis.

Furthermore, exemplary aspects and embodiments are further explained in relation to the appended Figures. It is noted that the exemplary embodiments shown in the Figures and further explained in the following are to be understood as illustrative, and thus non-limiting, only.

Specifically, the Figures show:

| | |
|---|---|
| **Figure 1:** | a perspective view from a forward-sidewards direction of a user wearing an exemplary user-wearable orthosis; |
| **Figure 2:** | a further perspective view from a rearward-sideward direction of the user wearing the exemplary user-wearable orthosis of Figure 1; |
| **Figure 3** | an exploded view of an exemplary proximal anchor unit; |
| **Figure 4:** | the exemplary proximal anchor unit of Figure 3 in an assembled state; |
| **Figure 5:** | an exemplary distal anchor unit in an exploded view; |
| **Figure 6:** | the distal anchor unit of Figure 5 in an assembled state; |
| **Figure 7:** | an exemplary embodiment of a common actuation unit; |
| **Figure 8:** | exemplary operational characteristics of the user-wearable orthosis of Figures 1 and 2; |
| **Figures 9A and 9B:** | exemplary separation of tasks between the pulling actuator and the adjustment actuator; |
| **Figure 10:** | an exemplary arrangement of the at least one sensor unit; |
| **Figure 11:** | an exemplary load sensor of the user-wearable orthosis; |
| **Figure 12:** | a schematic overview of a control method for the user-wearable orthosis; |
| **Figure 13:** | a flow diagram of an exemplary method for controlling a user-wearable orthosis for assisting a user motion of a joint of a user; |
| **Figure 14:** | a schematic illustration of exemplary joint motion data to be measured and/or determined by the at least one sensor unit; |
| **Figure 15:** | a schematic representation of the three rotations from the world frame to obtain the limb solidal frame; |
| **Figure 16:** | a schematic geometrical model of an arm while wearing an exemplary user-wearable orthosis; and |
| **Figure 17:** | a graph of a sigmoid interpolation between two values of the adaptive filter parameter $\lambda$ as a function of the elevation angle $\alpha$. |

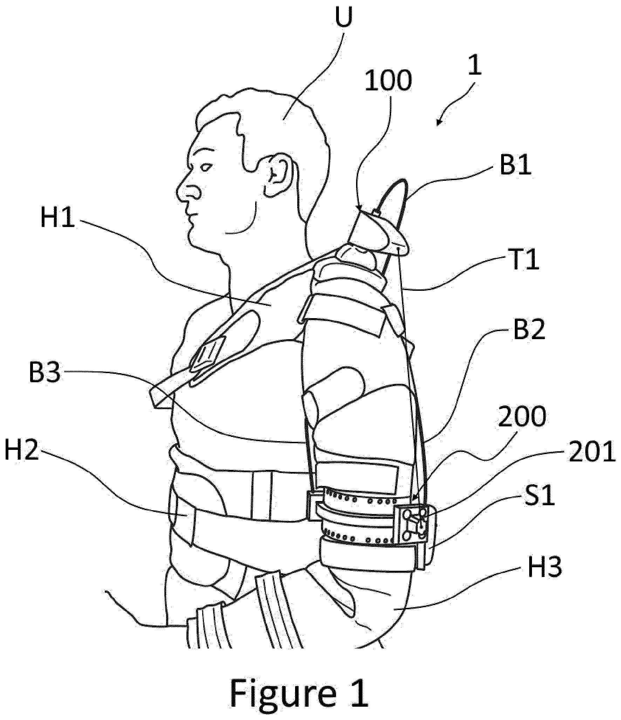

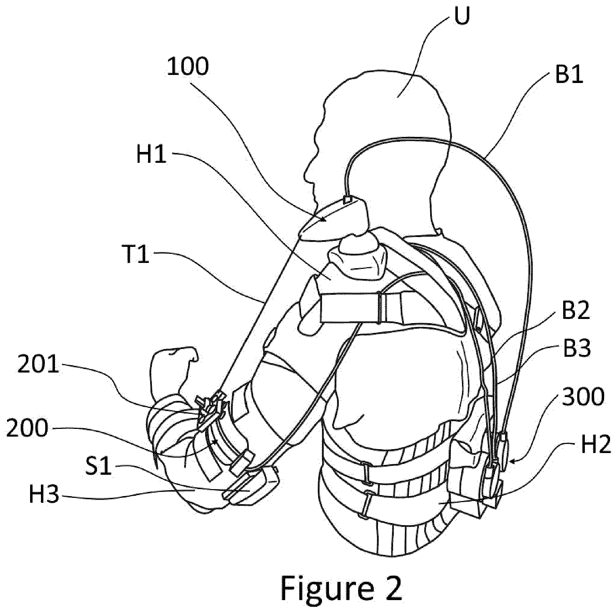

**Figure 1** shows a perspective view from a forward-sidewards direction of a user **U** wearing an exemplary user-wearable orthosis **1**. **Figure 2** shows a further perspective view from a rearward-sideward direction of the user **U** wearing the exemplary user-wearable orthosis **1**. As Figures 1 and 2 show different views of the same exemplary user-wearable orthosis **1,** both Figures 1 and 2 are described in parallel below.

The shown user-wearable orthosis **1** is implemented as a soft exosuit worn by the user **U,** wherein the exemplary user-wearable orthosis **1** is configured to assist a user motion of a joint of the user **U,** specifically the user motion of the (in this exemplary case left) shoulder joint of the user **U.** Furthermore, assistance to the user motion is given in and/or along at least the frontal plane of the user **U** (e.g., lateral or side-to-side motion during lifting of the left arm) and the sagittal plane of the user **U** (e.g., forward and backward motion during lifting of the left arm), thereby providing assistance to said user motion in two degrees of freedom.

In particular, the user-wearable orthosis **1** comprises a user-wearable harness having three harness elements. The harness elements comprise a shoulder harness element **H1** configured to be worn at least partially on a shoulder and/or on a shoulder joint of the user **U,** a torso harness element **H2** configured to be worn at least partially on a torso and/or waist of the user **U,** and an arm harness element **H3** configured to be worn at least partially on an upper arm and at least partially on a lower arm of the user **U.**

The user-wearable orthosis **1** comprises a proximal anchor unit **100** mountable on or proximal to the shoulder joint of the user **U.** The proximal anchor unit **100** is connectable to a force transfer element **T1** to partially guide the force transfer element **T1** between the distal anchor unit **200** and a pulling actuator (not directly shown in Figures 1 and 2) provided and/or housed in a common actuation unit **300.** Furthermore, the proximal anchor unit **100** is mountable on the shoulder harness element **H1.** The common actuation unit **300** is mountable on the torso harness element **H2.**

In the shown user-wearable orthosis **1,** the force transfer element **T1** is implemented as a tendon. The force transfer element **T1** further comprises a Bowden sheath **B1,** wherein the force transfer element **T1** is configured to extend at least partially within the Bowden sheath **B1.** Specifically, the Bowden sheath **B1** extends from the common actuation unit **300** to the proximal anchor unit **100.** The force transfer element **T1** extends from the common actuation unit **300** within the Bowden sheath **B1** to the proximal anchor unit **100.** The proximal anchor unit **100** partially guides the force transfer element **T1** towards the distal anchor unit **200,** wherein the force transfer element **T1** extends (without Bowden sheath **B1**) from the proximal anchor unit **100** to the distal anchor unit **200.** In particular, the force transfer element **T1** may be fixedly or releasably connected to an anchor point element **201** of the distal anchor unit **200.**

The user-wearable orthosis **1** further comprises the distal anchor unit **200** mountable on the (in this exemplary case left) arm of the user **U.** The distal anchor unit **200** is mountable on the arm harness element **H3.** The distal anchor unit **200** further comprises the anchor point element **201,** wherein the anchor point element **201** is moveable at least partially circumferentially along a circumferential direction around the arm of the user **U.** As shown in Figures 1 and 2, the distal anchor unit **200** is configured to partially surround the arm of the user **U** along the circumferential direction.

The user-wearable orthosis **1,** and specifically the pulling actuator, is configured to exert a pulling force on the force transfer element **T1** to assist the user motion of the shoulder joint. In particular, by exerting the pulling force, via the pulling actuator located in the common actuation unit **300,** on the force transfer element **T1** an arm lifting motion with two degrees of freedom may be assisted.

The user-wearable orthosis **1** further comprises a movement prediction unit (not shown for illustrative purposes) configured to at least partially predict the user motion of the joint. In addition, the user-wearable orthosis **1** further comprises an anchor point element adjustment unit configured to control a circumferential position of the anchor point element **201** around the arm based on the at least partially predicted user motion of the joint.

The anchor point element adjustment unit comprises an adjustment actuator (not shown in Figures 1 and 2) connectable, via an adjustment force transfer element (not explicitly shown for illustrative reasons), to the distal anchor unit **200** and the anchor point element **201.** The adjustment actuator is provided and/or housed in the common actuation unit **300** and further configured to exert an adjustment pulling force on the adjustment force transfer element.

In the shown exemplary user-wearable orthosis **1,** the adjustment force transfer element is implemented as a tendon. The adjustment force transfer element is guided within a first adjustment Bowden sheath **B2** and a second adjustment Bowden sheath **B3** between the distal anchor unit **200** and the common actuation unit **300.**

The adjustment actuator located in the common actuation unit **300** is configured to exert the adjustment pulling force on the adjustment force transfer element to control the circumferential position of the anchor point element **201.**

In the shown exemplary user-wearable orthosis **1,** the adjustment force transfer element is implemented as a circular tendon having a first end and a second end. The first end of the circular tendon is fixed to the anchor point element **201,** wherein the circular tendon extends from the first end at the anchor point element **201** at least partially along the distal anchor unit **200.** The distal anchor unit **200** is configured to guide the circular tendon towards the adjustment actuator in the common actuation unit **300,** in particular within the first adjustment Bowden sheath **B2.** The first adjustment Bowden sheath **B2** extends from the distal anchor unit **200** to the common actuation unit **300.** The circular tendon is connected to the adjustment actuator and further extends from the adjustment actuator back to the distal anchor unit **200** within the second adjustment Bowden sheath **B3.** In particular, the second adjustment Bowden sheath **B3** extends from the common actuation unit **300** to the distal anchor unit **200.** Both the first and second adjustment Bowden sheaths **B2, B3** may be at least partially fixed or fixable to the torso harness element **H2,** the shoulder harness element **H1,** and/or the arm harness element **H3.** The circular tendon may then be guided by the distal anchor unit **200** towards the anchor point element **201,** wherein the second end of the circular tendon is fixed to the anchor point element **201.**

The user-wearable orthosis **1** further comprises at least one sensor unit, such as for example IMU sensor **S1** mounted on the arm of the user **U,** as shown in Figures 1 and 2. In particular, IMU sensor **S1** is mounted on the arm harness element **H3.**

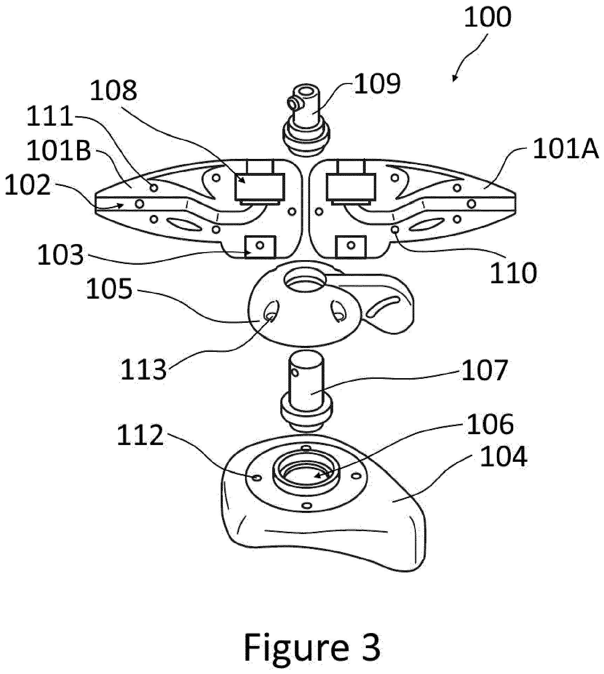

**Figure 3** shows an exploded view of an exemplary proximal anchor unit **100.**

The proximal anchor unit **100** comprises an anchor base mountable on the user **U,** specifically on the shoulder harness element **H1.** The anchor base comprises at least a first base element **104** and a second base element **105.** The first base element **104** is configured to be mountable on the shoulder harness element **H1.**

The second base element **105** is fixedly or releasably connectable to the first base element **104.** For example, the first base element **104** may comprise at least one first fixation bore **112** and the second base element **105** may comprise at least one second fixation bore **113,** wherein each first fixation bore **112** is configured to be aligned with and/or correspond to one of the second fixation bores **113.** The second base element **105** may be releasably connectable to the first base element **104** by tightening one or more fixation screws into the first and second fixation bores **112, 113.**

Furthermore, the first base element **104** and the second base element **105** define therebetween a base cavity **106.** The base cavity **106** is configured to at least partially receive and/or accommodate the rotational bearing **107** further described below. In particular, the base cavity **106** is configured to allow substantially free rotation of the rotational bearing **107** relative to the anchor base. Furthermore, the base cavity **106,** such as the exemplary base cavity **106** shown in Figure 3, may be configured to prevent a movement of the rotational bearing **107** along the first rotation axis after the first and second base elements **104, 105** have been fixedly or releasably connected to one another.

The proximal anchor unit **100** further comprises a force transfer element guiding device **101** configured to partially guide the force transfer element **T1** towards the anchor point element **201**. In particular, the force transfer element guiding device **101** comprises a guiding cavity **102** extending at least partially through the force transfer element guiding device **101**, wherein the guiding cavity **102** is configured to at least partially guide and/or accommodate the force transfer element **T1**.

The force transfer element guiding device **101** comprises a first guiding element **101A** and a second guiding element **101B**. The first guiding element **101A** and the second guiding element **101B** are configured to be releasably connectable to one another. In particular, the first guiding element **101A** may comprise at least one third fixation bore **110** and the second guiding element **101B** may comprise at least one fourth fixation bore **111**. Each third fixation bore **110** is configured to be aligned with and/or correspond to one of the fourth fixation bores **111**. The first guiding element **101A** may be releasably connectable to the second guiding element **101B** by tightening one or more fixation screws into the third and fourth fixation bores **110, 111**.

In particular, the first guiding element **101A** and the second guiding element **101B** define therebetween the guiding cavity **102**.

Furthermore, the first guiding element **101A** and the second guiding element **101B** define therebetween a guiding element base cavity **103**. The guiding element base cavity **103** is configured to partially receive and/or accommodate the rotational bearing **107**. In addition, the rotational bearing **107** is fixedly connectable to the force transfer element guiding device **101**, such as fixedly connectable to the guiding element base cavity **103**. For example, a rotational bearing fixation element, such as a screw or bolt, may be provided to fixedly connect the rotational bearing **107** to the force transfer element guiding device **101** and/or to the guiding element base cavity **103**.

The proximal anchor unit **100** further comprises the rotational bearing **107** provided between the anchor base and the force transfer element guiding device **101**. The rotational bearing **107** is configured to allow the force transfer element guiding device **101** to substantially freely rotate around a first rotation axis relative to the anchor base in response to a change in the circumferential position of the anchor point element **201** around the arm. In the shown exemplary embodiment, the rotational bearing **107** is implemented as a shaft configured to fixedly connect to the force transfer element guiding device **101**, wherein the shaft is configured to substantially freely rotate with respect to the anchor base.

The proximal anchor unit **100** further comprises a Bowden sheath abutment device **109** configured to be connectable to at least one end of the Bowden sheath **B1** of the force transfer element **T1**. The one end of the Bowden sheath **B1** may freely abut the Bowden sheath abutment device **109**. Alternatively, the one end of the Bowden sheath **B1** may be fixedly or releasably mountable to the Bowden sheath abutment device **109**. The Bowden sheath abutment device **109** may comprise a lateral opening perpendicular to its axis, which may be used to place a screw and block the one end of the Bowden sheath **B1** in position.

The Bowden sheath abutment device **109** is further connectable to the force transfer element guiding device **101**, wherein the Bowden sheath abutment device **109** and the force transfer element guiding device **101** are configured to be substantially freely rotatable relative to one another around a second rotation axis in response to the change in the circumferential position of the anchor point element around the arm. Specifically, the first guiding element **101A** and the second guiding element **101B** define therebetween an abutment cavity **108** configured to partially receive and/or accommodate the Bowden sheath abutment device **109**. The abutment cavity **108** is configured to allow said substantially free rotation of the Bowden sheath abutment device **109** relative to the force transfer element guiding device **101**.

In particular, the first rotation axis is substantially identical to the second rotation axis to allow for a very efficient and facile operation of the user-wearable orthosis **1**.

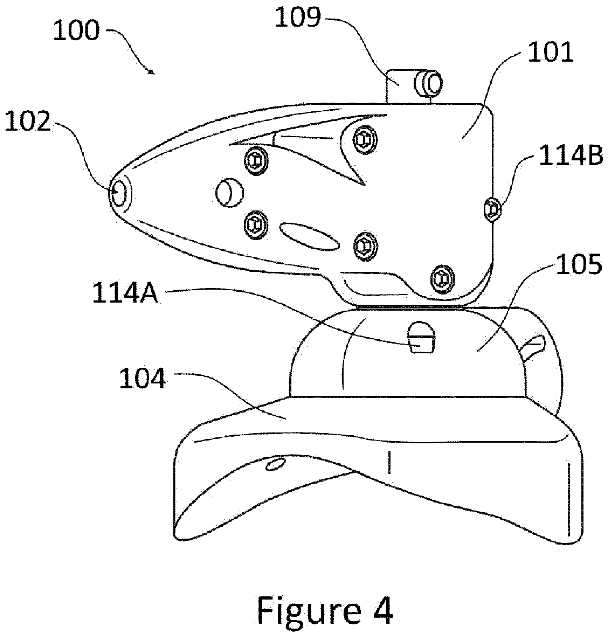

**Figure 4** shows the exemplary proximal anchor unit **100** of Figure 3 in an assembled state.

As shown in Figure 4, the second base element **105** is releasably connected to the first base element **104** by tightening one or more fixation screws **114A** into the first and second fixation bores **112, 113**. Similarly, the first guiding element **101A** is releasably connected to the second guiding element **101B** by tightening one or more fixation screws **114B** into the third and fourth fixation bores **110, 111**.

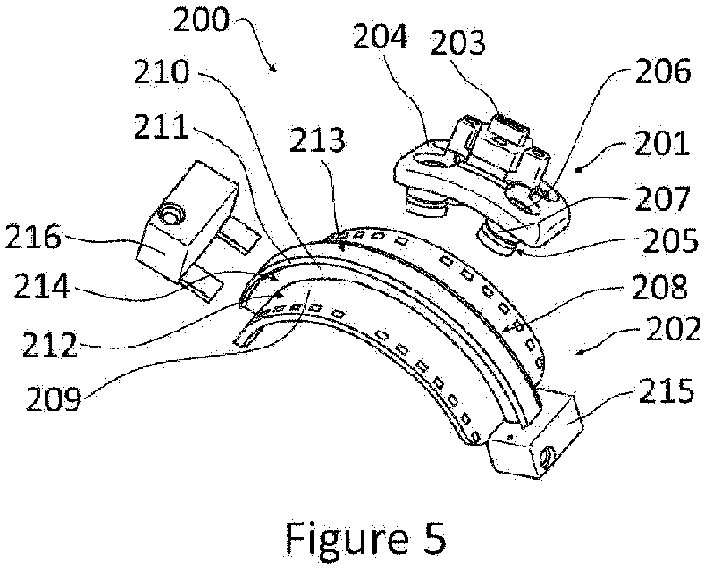

**Figure 5** shows an exemplary distal anchor unit **200** in an exploded view. The shown distal anchor unit **200** comprises an anchor point element **201** and a guidance element **202**.

Specifically, the distal anchor unit **200** comprises the anchor point element **201** moveable at least partially circumferentially along a circumferential direction around the arm of the user **U**. The distal anchor unit **200** is configured to partially surround the arm of the user **U** along the circumferential direction.

The anchor point element **201** comprises a force transfer element connection point **203**, wherein the force transfer element **T1** may be fixedly or releasably connectable to the force transfer element connection point **203**. The anchor point element **201** further comprises a movement base element **204**. The movement base element **204** may be configured as a cart and/or sled. The exemplary movement base element **204** shown in Figure 5 comprises four wheel elements **205** fixedly connected to the movement base element **204**. In particular, each wheel element **205** comprises a wheel axis **206** and a wheel **207**, wherein the wheel axis **206** is fixedly connected to the movement base element **204**, and wherein the wheel **207** is rotatable around the wheel axis **206**.

The four wheel elements **205** are configured to be engageable with the guidance element **202,** wherein each wheel **207** of the four wheel elements **205** is configured to enter into contact with the guidance element **202.** Specifically, each wheel **207** is configured to roll along the guidance element **202** during movement of the anchor point element **201** along the guidance element **202** and/or the circumferential direction. Furthermore, two of the four wheel elements **205** are provided to engage the first rib side surface (described below) and the two other wheel elements **205** are provided to engage the second rib side surface (described below).

The distal anchor unit **200** further comprises the guidance element **202** configured to partially circumferentially surround the arm of the user **U.** In the shown exemplary embodiment, the guidance element **202** comprises a guide rail **208.** The guidance element **202** is configured to allow the anchor point element **201** to move circumferentially around the arm within the circumferential range, while preventing and/or restricting movement of the anchor point element **201** in other directions.

In the shown example, the guidance element **202** comprises a base plate **209** configured to partially surround the arm of the user **U** in the circumferential direction. In particular, the base plate **209** is configured to be mountable on the arm harness element **H3.**

The guide rail **208** comprises at least a first guide rail element **210** and a second guide rail element **211.** Both the first guide rail element **210** and the second guide rail element **211** are configured to guide the anchor point element **201** along the circumferential direction. The first guide rail element **210** is a rib element extending along an outer surface **212** of the base plate **209** in the radial direction. The first guide rail element **210** further extends along a length of the base plate **209.** Specifically, the first guide rail element **210** comprises an upper rib surface **213** substantially parallel to the outer surface **212,** a first rib side surface **214,** and a second rib side surface (not shown for perspective reasons).

The second guide rail element **211** is a circumferential protrusion protruding from the first rib side surface **214.** The second guide rail element **211** further extends substantially along the first rib side surface **214** along the circumferential direction, and is arranged abutting the upper rib surface **213** of the first guide rail element **210.**

The guide rail **208** may further comprise a third guide rail element. However, for illustrative reasons, said third guide rail element is not shown in Figure 5. In particular, the second guide rail element **211** and the third guide rail element are positioned opposite one another with respect to the first guide rail element **210.**

The base plate **209** and the guide rail **208,** comprising at least the first guide rail element **210** and the second guide rail element **211,** are formed integrally.

The guidance element **202** further comprises a first stopping section **215** positioned at a first end of the guidance element **202** along the circumferential direction and a second stopping section **216** at a second end of the guidance element **202** along the circumferential direction. In particular, the first stopping section **215** and the second stopping section **216** are configured to prevent the anchor point element **201** from dropping off the guidance element **202** at the respective first end and second end.

In the shown exemplary embodiment, the first stopping section **215** is integrally formed with the guide rail **208** and the base plate **209,** while the second stopping section **216** is detachable from guide rail **208** and the base plate **209.**

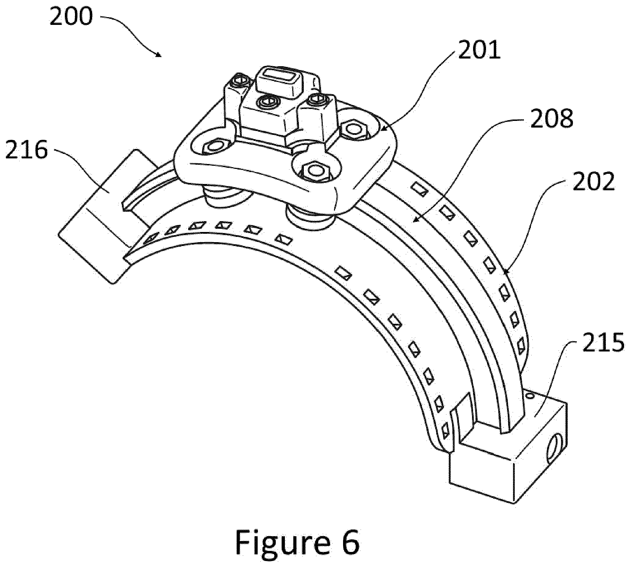

**Figure 6** shows the distal anchor unit **200** of Figure 5 in an assembled state.

In particular, the anchor point element **201** is guided by the guide rail **208** of the guidance element **202** and configured to be movable along said guide rail **208** in the circumferential direction. The anchor point element **201** is prevented from falling off or dropping off the guide rail **208** at its first and second ends in the circumferential direction by the respective first and second stopping sections **215, 216.**

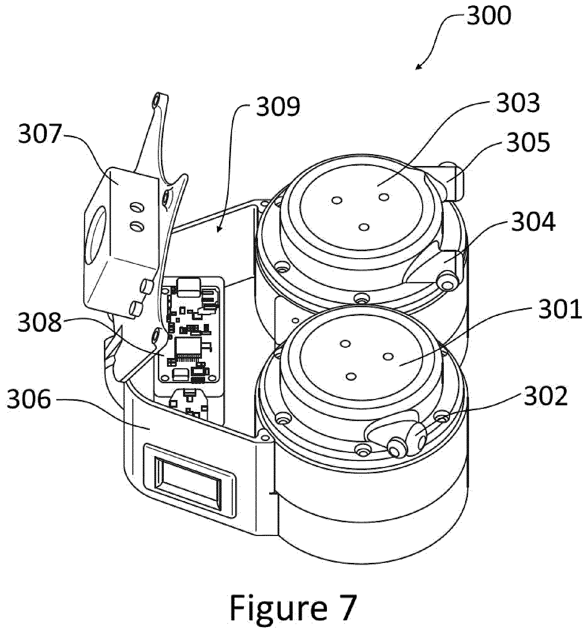

**Figure 7** shows an exemplary embodiment of a common actuation unit **300.**

In particular, the user-wearable orthosis **1** and/or the common actuation unit **300** comprise the pulling actuator **301** configured to exert the pulling force on the force transfer element **T1.** In the shown example, the pulling actuator **301** is configured as a rotary actuator connected to the force transfer element **T1.** A specific exemplary pulling actuator **301** may be an AK60-6 rotary actuator.

The pulling actuator **301** further comprises a pulling actuator abutment section **302** configured to be connectable to an end of the Bowden sheath **B1** of the force transfer element **T1.** Said end of the Bowden sheath **B1** may freely abut the pulling actuator abutment section **302** or be fixedly or releasably mountable to the pulling actuator abutment section **302.** Furthermore, the pulling actuator abutment section **302** may further be configured to guide the force transfer element **T1** from the Bowden sheath **B1** into the pulling actuator **301,** such as to a spool (not shown) of the pulling actuator **301.**

The user-wearable orthosis **1** and/or the common actuation unit **300** further comprise the adjustment actuator **303** connectable, via an adjustment force transfer element, to the anchor point element **201.** The adjustment actuator **303** is configured to exert the adjustment pulling force on the adjustment force transfer element to control the position of the anchor point element **201** in the circumferential direction.

The adjustment actuator **303** further comprises a first adjustment actuator abutment section **304** configured to be connectable to an end of the first adjustment Bowden sheath **B2** of the adjustment force transfer element. Said end of the first adjustment Bowden sheath **B2** may freely abut the first adjustment actuator abutment section **304** or be fixedly or releasably mountable to the first adjustment actuator abutment section **304.** Furthermore, the first adjustment actuator abutment section **304** may be configured to guide the adjustment force transfer element from the first adjustment Bowden sheath **B2** into the adjustment actuator **303** (and vice versa), such as to a spool (not shown) of the adjustment actuator **303**.

The adjustment actuator **303** further comprises a second adjustment actuator abutment section **305** configured to be connectable to an end of the second adjustment Bowden sheath **B3** of the adjustment force transfer element. Said end of the second adjustment Bowden sheath **B3** may freely abut the second adjustment actuator abutment section **305** or be fixedly or releasably mountable to the second adjustment actuator abutment section **305**. Furthermore, the second adjustment actuator abutment section **305** may be configured to guide the adjustment force transfer element from the second adjustment Bowden sheath **B3** into the adjustment actuator **303** (and vice versa), such as to a spool (not shown) of the adjustment actuator **303**.

The adjustment actuator **303** may be any kind of actuator capable of exerting, directly or indirectly, the adjustment pulling force on the adjustment force transfer element. The exemplary, shown adjustment actuator **303** is a rotary actuator, such as an AK60-6 rotary actuator.

By providing the common actuation unit **300** comprising the pulling actuator **301** and the adjustment actuator **303**, a separation of tasks for the user-wearable orthosis **1** for assisting the user motion may be achieved. In particular, the pulling actuator **301** may be configured to exert the pulling force necessary to provide the assistance to the user motion, while the adjustment actuator **303** may be configured to exert the adjustment pulling force necessary for moving the anchor point element **201** to the correct/desired location in the circumferential direction.

Both the adjustment actuator **303** and the pulling actuator **301** are provided and/or housed in the common actuation unit **303**, specifically in a housing **306** of the common actuation unit **300**. Furthermore, the common actuation unit **300** and/or the housing **306** may be configured to be mountable on the torso harness element **H2**.

The housing **306** further comprises a hinged lid **307**, wherein the hinged lid **307** is configured to be moveable between a closed state and an opened state. The housing **306** and the hinged lid **307** further define an internal space **309** configured to house a control unit **308**.

The control unit **308** may in particular comprise at least the movement prediction unit. An exemplary control unit **308** may be an Arduino MKR1010WiFi, wherein the control unit **308** may be connected, e.g., via wired or wireless connection (for example via CAN-bus), to the pulling actuator **301** and/or the adjustment actuator **303** and/or the at least one sensor unit, such as IMU sensor **S1**.

The control unit **308** may be configured to receive data input from, provide data output to, and/or control operation of multiple parts of the user-wearable orthosis **1**. For example, the control unit **308** may comprise a pulling actuator control unit configured to control the operation of the pulling actuator **301** and/or an adjustment actuator control unit configured to control the operation of the adjustment actuator **303**.

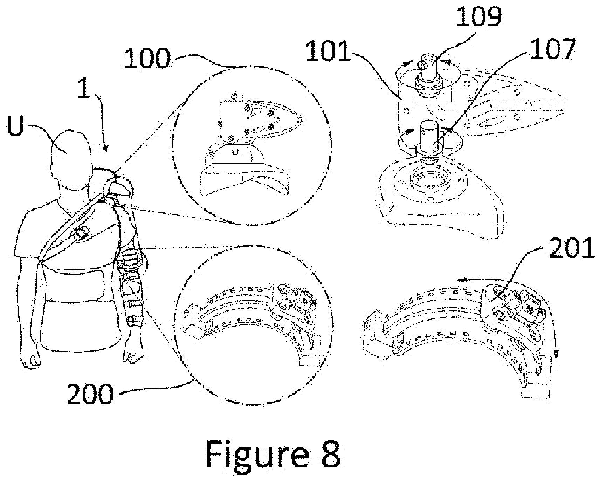

**Figure 8** illustrates exemplary operational characteristics of the user-wearable orthosis **1** of Figures 1 and 2. Specifically, the user-wearable orthosis **1** is worn by the user **U**.

By providing a proximal anchor unit **100** having both a rotational bearing **107** and a Bowden sheath abutment device **109**, it becomes possible to decouple a rotational orientation of the force transfer element guiding device **101** from a rotational state of the Bowden sheath **B1** and a rotational state of the shoulder and a rotational state of the shoulder harness element **H1**. In other words, rotational bearing **107** and Bowden sheath abutment device **109** may form a double revolute joint such that the force transfer element guiding device **101** is capable of rotating around the first and second rotation axis independently of the Bowden sheath **B1**, the shoulder, and the shoulder harness element **H1**. The force transfer element guiding device **101** may therefore passively rotate around the first and second rotation axis according to a circumferential position of the anchor point element **201**.

Furthermore, by providing a distal anchor unit **200** having a guidance element **202** and the anchor point element **201**, it becomes possible to control a circumferential position of the anchor point element **201** using a corresponding adjustment actuator **303**. Please note that while the exemplary adjustment actuator **303** is shown as being housed in the common actuation unit **300**, the adjustment actuator **303** may be arranged and/or positioned elsewhere, such as for example in the distal anchor unit **200**, the proximal anchor unit **100**, and/or the anchor point element **201**.

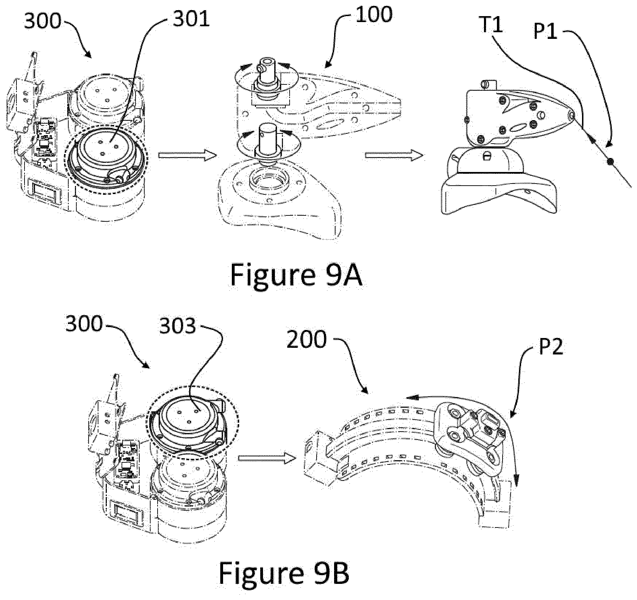

**Figures 9A and 9B** illustrate the exemplary separation of tasks between the pulling actuator **301** and the adjustment actuator **303**.

In Figure 9A, the pulling actuator **301** in the common actuation unit **300** is configured to exert the pulling force **P1** on the force transfer element **T1**, wherein the force transfer element **T1** is guided by the proximal anchor unit **100**, specifically by the force transfer element guiding device **101** of the proximal anchor unit **100** towards the distal anchor unit **200**. The pulling actuator **301** may therefore be implemented as a high-power actuator configured to act as a gravity compensation mechanism for the arm of the user **U** during lifting of said arm, thereby assisting the user motion.

In Figure 9B, the adjustment actuator **303** in the common actuation unit **300** is configured to exert the adjustment pulling force **P2** via the adjustment force transfer element on the anchor point element **201**, thereby allowing a control of the circumferential position of the anchor point element **201** of the distal anchor unit **200**. The adjustment actuator **303** may therefore be implemented as a low-power actuator configured to cause the anchor point element **201** to move in one or two directions along the circumferential direction.

Thus, while the pulling actuator **301** may be configured to provide active assistance to the user motion, the adjustment actuator **303** may be configured to provide active alignment of the anchor point element **201** around the arm of the user **U** according to the desired and/or predicted user motion.

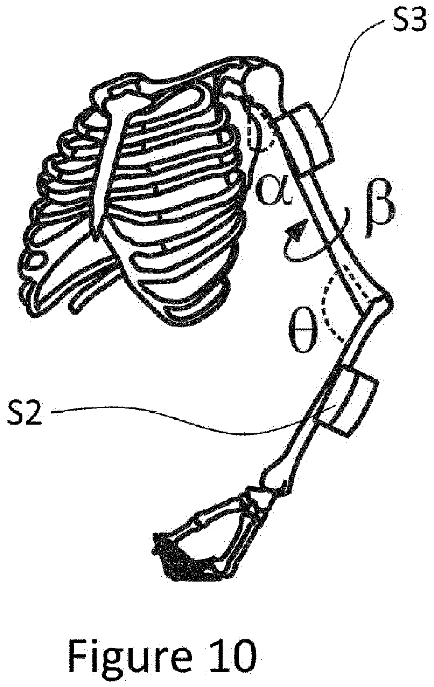

**Figure 10** shows an exemplary arrangement of the at least one sensor unit. Specifically, the at least one sensor unit comprises a first IMU sensor **S2** arranged on a lower arm of the user **U** and a second IMU sensor **S3** arranged on an upper arm of the user **U.**

The first IMU sensor **S2** and the second IMU sensor **S3** are configured to measure a 3D orientation of the body of the user U, an elevation angle $\alpha$ of the shoulder, a longitude angle $\beta$, and an elbow flexion angle $\theta_e$. The parameters measured by the first IMU sensor **S2** and the second IMU sensor **S3** may be used to control the user-wearable orthosis **1.**

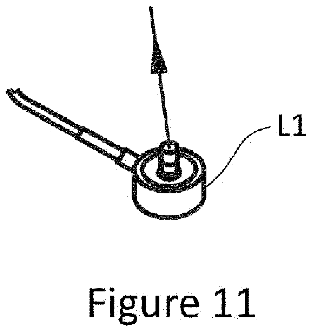

**Figure 11** shows an exemplary load sensor of the user-wearable orthosis **1.**

In particular, the user-wearable orthosis **1** may comprise a load sensor to measure the pulling force exerted by the pulling actuator **301** and/or a tension of the force transfer element **T1.**

Specifically, the load sensor may be implemented as a load cell **L1,** wherein the load cell **L1** is located between the force transfer element **T1** and the anchor point element **201,** such as between the force transfer element **T1** and the force transfer element connection point **203.**

**Figure 12** shows a schematic overview of a control method for the user-wearable orthosis **1.**

The exemplary control method, as shown in Figure 12, may be separated into an upper branch (labelled "Gravity Compensation Algorithm" in Figure 12), representing an operation of a pulling actuator control unit, and a lower branch (labelled "Reconfiguration Algorithm" in Figure 12), representing an operation of an adjustment actuator control unit.

At least one sensor unit may be configured to measure the elevation angle $\alpha$, the longitude angle $\beta$, and the elbow flexion angle $\theta_e$. A load cell may be provided to measure a tension $f$ in the force transfer element.

The pulling actuator control unit may be configured to determine, using for example a GTE module (labelled as "Dynamic Arm Module" in Figure 12), a gravitational compensation torque $\tau_g$. Specifically, the GTE module may be configured to receive as input the elevation angle $\alpha$, the longitude angle $\beta$, and the elbow flexion angle $\theta_e$, and to determine, based on such an input, the gravitational compensation torque $\tau_g$, as discussed in relation to the GTE module above. The GTE module may be configured to output the gravitational compensation torque $\tau_g$, labelled as reference torque $\tau_r$, as shown in Figure 12.

The pulling actuator control unit may be configured to estimate, using for example an ATE module (labelled as "Moment Arm" in Figure 12), a torque applied by the pulling actuator.

Specifically, the ATE module may be configured to receive, as input, the elevation angle $\alpha$ and the tension $f$ in the force transfer element. The ATE module may be further configured to, based on such an input, estimate the torque applied by the pulling actuator, as discussed in relation to the ATE module above. The ATE module may be configured to output the estimated torque, labelled as interaction torque $\tau_i$ in Figure 12.

The pulling actuator control unit may be configured to determine an error torque $\tau_{error}$ between the reference torque $\tau_r$ and the interaction torque $\tau_i$.

The pulling actuator control unit may be further configured to translate, using for example an admittance control module (labelled "Admittance Control" in Figure 12), the torque error $\tau_{error}$ into a velocity command for the pulling actuator. The admittance control module may be configured, as discussed above. In particular, the velocity command may be output to the pulling actuator to cause the pulling actuator to exert the pulling force on the force transfer element.

In particular, the motion prediction unit may be configured to receive the elevation angle $\alpha$ and the longitude angle $\beta$, and may be further configured to apply an exponential moving average (EMA) adaptive filter, as a function of the elevation angle $\alpha$, to the longitude angle $\beta$. The EMA adaptive filter may be implemented as discussed above.

Therefore, the motion prediction unit may be configured to apply an EMA adaptive filter, as a function of the elevation angle $\alpha$, to the longitude angle $\beta$ in order to obtain a filtered longitude angle $\beta_{filt}$. The filtered longitude angle $\beta_{filt}$ may be output to the anchor point element adjustment unit.

The anchor point element adjustment unit may be configured to receive both the filtered longitude angle $\beta_{filt}$ and a feedback motor position $\beta_{fb}$. The feedback motor position $\beta_{fb}$ may correspond to a current motor position of the adjustment actuator, wherein the adjustment actuator may be configured to output said feedback motor position $\beta_{fb}$ to the anchor point element adjustment unit.

The anchor point element adjustment unit may then be configured to determine a difference $\beta_{error}$ between the filtered longitude angle $\beta_{filt}$ and the feedback motor position $\beta_{fb}$, wherein said difference $\beta_{error}$ may be output to the adjustment PID position controller in order to control the position of the anchor point element.

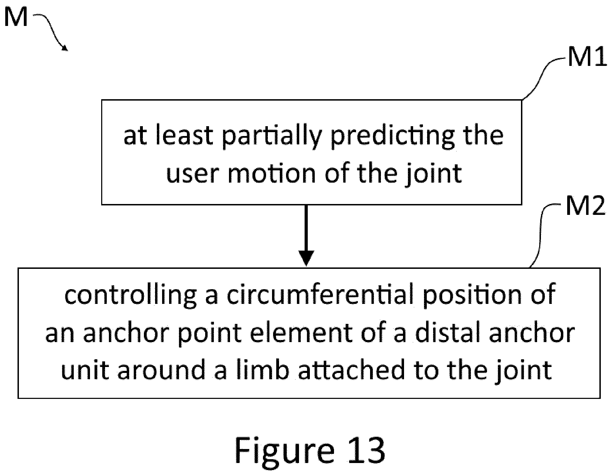

**Figure 13** shows a flow diagram of an exemplary method **M** for controlling a user-wearable orthosis **1** for assisting a user motion of a joint of a user **U.**

The method **M** comprises a step **M1** of at least partially predicting the user motion of the joint.

In a further step, the method comprises a step of controlling a circumferential position of an anchor point element **201** of a distal anchor unit **200** around a limb attached to the joint, such as around an arm attached to a shoulder joint, based on the at least partially predicted user motion of the joint.

In particular, the distal anchor unit **200** may be mounted on the limb, wherein a proximal anchor unit **100** mounted on or proximal to the joint is connectable to the anchor point element **201** via a force transfer element **T1,** wherein the user-wearable orthosis **1** is configured to exert a pulling force **P1** on the force transfer element **T1** to assist the user motion.

Furthermore, the anchor point element **201** is moveable at least partially circumferentially around the limb of the user **U.**

**Figure 14** shows a schematic illustration of exemplary joint motion data to be measured and/or determined by the at least one sensor unit.

Specifically, the exemplary joint motion data may comprise at least one of a current elevation angle $\alpha$ of the limb, represented as an upper arm in the shown example, and a longitude angle $\beta$ of the limb.

The elevation angle $\alpha$ may be defined as the angle between a z-axis $Z_w$ of the world frame **<w>** and a z-axis $Z_u$ of the limb solidal frame **<u>.** In particular, the z-axis $Z_u$ of the limb solidal frame **<u>** may be parallel to the limb axis of the upper arm shown. Therefore, the elevation angle $\alpha$ may be indicative of a current elevation of the limb of the user, e.g., of the upper arm of the user.

Figure 14 further shows an exemplary longitude angle $\beta$ and illustrates how said exemplary longitude angle $\beta$ may be defined. A circumference may be defined such that said circumference is centred on an origin of the limb solidal frame **<u>,** as shown. Said circumference may be constituted by a set of points having coordinates in the world frame **<w>.** A highest point may be defined as a point in said set of points having a highest value z-coordinate in the world frame **<w>** (indicated as "max" in Figure 14). A first line may be defined to extend through the highest point and the origin of the limb solidal frame **<u>.** The longitude angle $\beta$ may be defined as the angle between the first line and the y-axis $Y_u$ of the limb solidal frame **<u>.** Please note that the present disclosure is not limited to such a determination of the longitude angle $\beta$. For example, the longitude angle $\beta$ may also be defined by replacing the highest point with the lowest point, which may be defined as a point in said set of points having a lowest value z-coordinate in the world frame **<w>.**

Furthermore, an orientation of a limb, such as the upper arm of the user, may be described using a fixed XYZ Euler angle parametrisation. Such a representation may involve three consecutive rotations of the world frame **<w>** around its initial axes: a first rotation by an angle $\theta_1$ around the X-axis, a second rotation by an angle $\theta_2$ around the Y axis, and a third rotation by an angle $\theta_3$ around the Z axis. Angles $\theta_1$, $\theta_2$, and $\theta_3$ may be the Euler angles. Such rotations are illustratively shown in Figure 15. Please note that while frames **<1>, <2>,** and **<3>** are depicted at a distance for illustrative clarity, they share the same origin.

Thus, **Figure 15** may schematically represent the three rotations from the world frame **<w>** to obtain the local frame, e.g., the limb solidal frame **<u>,** which may represent the orientation of the shoulder in the space from the data of the sensor unit, e.g., the at least one IMU sensor, or may represent the orientation of the lower arm in the space from the data of the sensor unit.

Furthermore, the arm of the user may be treated as a kinematic chain consisting of rigid bodies interconnected by joints. The hand may be assumed to be rigidly attached to the lower arm (forearm), disregarding the wrist joint. Finally, the shoulder joint may be simplified to have three degrees of freedom.

The equations of motion for a conventional kinematic chain may be obtained using the Lagrange method. Considering $K$ as the total kinetic energy of the system and $U$ as the total potential energy, the overall energy can be expressed as $L = K - U$. Then, if $\tau_i$ represents the element of the generalized force vector corresponding to the joint configuration variable $q_i$, the Lagrangian dynamic equations may be as follows (equation 15.1):

$$\frac{d}{dt}\frac{\partial L}{\partial \dot{q}_i} - \frac{\partial L}{\partial q_i} = \tau_i; \quad i = 1,2,3,4$$

Furthermore, the above equation may take the following form (equation 15.2):

$$\mathbf{M}(\mathbf{q})\ddot{q}(t) + \mathbf{C}(\mathbf{q},\dot{\mathbf{q}})\dot{q}(t) + \mathbf{G}(\mathbf{q}) = \boldsymbol{\tau}$$

where:

$\ddot{q}$, $\dot{q}$, $q$ correspond to the joint accelerations, joint velocities, and joint positions, respectively;
**M(q)** corresponds to a joint space mass matrix or a generalised inertia matrix;
**C(q,q̇)** corresponds to a matrix accounting for Coriolis and centrifugal effects;
**G(q)** corresponds to a gravity compensation torque; and
$\tau$ corresponds to a vector of overall torques applied at the joints.

Furthermore, it may be assumed that movements of the arm are typically slow and smooth, translating into low joint accelerations and velocities. Therefore, equation 15.2 may be approximated in quasi-static conditions as (equation

15.3):

$$G(q) \approx \tau$$

Given equations 15.1 and 15.2, the term G(q) may be given by (equation 15.4):

$$G(q) = -\frac{d}{dt}\frac{\partial U}{\partial \dot{q}} + \frac{\partial U}{\partial q}$$

In addition, a mapping between the XYZ Euler angle representation between the elevation angle α of the limb and the longitude angle β of the limb may be determined.

Specifically, a rotation matrix ${}^{0}_{3}R$ corresponding to the rotations corresponding to the Euler angles may be given by (equation 15.5):

$$
{}^{0}_{3}R = \begin{bmatrix} c(\theta_3) & -s(\theta_3) & 0 \\ s(\theta_3) & c(\theta_3) & 0 \\ 0 & 0 & 1 \end{bmatrix} * \begin{bmatrix} c(\theta_2) & 0 & s(\theta_2) \\ 0 & 1 & 0 \\ -s(\theta_2) & 0 & c(\theta_2) \end{bmatrix} * \begin{bmatrix} 1 & 0 & 0 \\ 0 & c(\theta_1) & -s(\theta_1) \\ 0 & s(\theta_1) & c(\theta_1) \end{bmatrix}
$$

$$
= \begin{bmatrix} c(\theta_3)c(\theta_2) & c(\theta_3)s(\theta_2)s(\theta_1) - s(\theta_3)c(\theta_1) & c(\theta_3)s(\theta_2)c(\theta_1) + s(\theta_3)s(\theta_1) \\ s(\theta_3)c(\theta_2) & s(\theta_3)s(\theta_2)s(\theta_1) + c(\theta_3)c(\theta_1) & s(\theta_3)s(\theta_2)c(\theta_1) - c(\theta_3)s(\theta_1) \\ -s(\theta_2) & c(\theta_2)s(\theta_1) & c(\theta_2)c(\theta_1) \end{bmatrix}
$$

Please note that, in order to maintain brevity, c has been used as shorthand for *cosine* and s has been used for *sine*.

Furthermore, the above rotation matrix (see equation 15.5) describes a rotation of the limb solidal frame <u> with respect to the world frame <w>, and thus will also be referred to as ${}^{w}_{u}R$ .

In addition, the solidal z-axis unit vector projected on the solidal frame may be given by (equation (15.6):

$$
{}^{u}\hat{k}_u = \begin{bmatrix} 0 \\ 0 \\ 1 \end{bmatrix}
$$

Said unit vector may be projected onto the world frame (equation 15.7):

$$
{}^{w}\hat{k}_u = {}^{w}_{u}R \begin{bmatrix} 0 \\ 0 \\ 1 \end{bmatrix} = \begin{bmatrix} c(\theta_3)s(\theta_2)c(\theta_1) + s(\theta_3)s(\theta_1) \\ s(\theta_3)s(\theta_2)c(\theta_1) - c(\theta_3)s(\theta_1) \\ c(\theta_2)c(\theta_1) \end{bmatrix}
$$

A scalar product of the above resulting unit vector (eq. 15.7) and the world frame z-axis unit vector (corresponding, per definition, to the cosine of the elevation angle $\alpha$) may be given by (equation 15.8):

$$
c(\alpha) = {}^{w}\hat{k}_u \cdot {}^{w}\hat{k}_w = \begin{bmatrix} c(\theta_3)s(\theta_2)c(\theta_1) + s(\theta_3)s(\theta_1) \\ s(\theta_3)s(\theta_2)c(\theta_1) - c(\theta_3)s(\theta_1) \\ c(\theta_2)c(\theta_1) \end{bmatrix} \begin{bmatrix} 0 & 0 & 1 \end{bmatrix} = c(\theta_2)c(\theta_1)
$$

Therefore, if the elevation angle $\alpha$ belongs to the interval $[0, \pi]$, the elevation angle $\alpha$ may be given as a function of the first and second Euler angles (equation 15.9):

$$
\alpha = acos\big(c(\theta_2)c(\theta_1)\big)
$$

Furthermore, the solidal y-axis may be chosen as the axis of the limb solidal frame with respect to which the longitude angle β of the limb may be evaluated. A circumference may be parameterised to have unitary radius, be centred on the origin of the limb solidal frame, and be projected onto the limb solidal frame (equation 15.10):

$$^{u}P_{u} = \begin{bmatrix} s(\eta) \\ c(\eta) \\ 0 \end{bmatrix}$$

Said parametrisation may be projected on the world frame (equation 15.11):

$$^{w}P_{u} = {}^{w}_{u}R \begin{bmatrix} s(\eta) \\ c(\eta) \\ 0 \end{bmatrix} = \begin{bmatrix} s(\eta)c(\theta_3)c(\theta_2) + c(\eta)[c(\theta_3)s(\theta_2)s(\theta_1) - s(\theta_3)c(\theta_1)] \\ s(\eta)s(\theta_3)c(\theta_2) + c(\eta)[s(\theta_3)s(\theta_2)s(\theta_1) + c(\theta_3)c(\theta_1)] \\ -s(\eta)s(\theta_2) + c(\eta)c(\theta_2)s(\theta_1) \end{bmatrix}$$

The z-component of the parametrised point of the circumference projected on the world frame may be extracted (equation 15.12):

$$^{w}P_{u}^{z} = -s(\eta)s(\theta_2) + c(\eta)c(\theta_2)s(\theta_1)$$

A partial derivative of said z-component with respect to $\eta$ may be given by (equation 15.13):

$$\frac{\partial\, ^{w}P_{u}^{z}}{\partial \eta} = -c(\eta)s(\theta_2) - s(\eta)c(\theta_2)s(\theta_1)$$

A value $\tilde{\eta}$, for which the z-component is maximal (or minimal), may be determined by solving the following equation (equation 15.14):

$$-c(\tilde{\eta})s(\theta_2) - s(\tilde{\eta})c(\theta_2)s(\theta_1) = 0$$

Therefore, the following equation may be arrived at (equation 15.15):

$$\frac{s(\tilde{\eta})}{c(\tilde{\eta})} = tan(\tilde{\eta}) = -\frac{s(\theta_2)}{c(\theta_2)s(\theta_1)}$$

It is noted that, by definition, $\tilde{\eta}$ coincides with the longitude angle $\beta$. Furthermore, if $\tilde{\eta}$ belongs to the interval [-π/2, π/2], it may be given by (equation 15.16):

$$\tilde{\eta} = atan\left(-\frac{s(\theta_2)}{c(\theta_2)s(\theta_1)}\right) = \beta$$

Based on equations 15.9 and 15.16, it may be observed that neither the elevation angle $\alpha$ nor the longitude angle $\beta$ depend on the third Euler angle $\theta_3$.

Thus, potential rotations of the limb solidal frame around the z-axis of the world frame, which may be very common and do not necessarily depend on the shoulder joint, do not affect the mapping. This, however, allows for a further simplification of the hardware of the user-wearable orthosis: a further sensor unit, e.g., a further IMU sensor, designed and configured to determine, measure, and/or derive $\theta_3$ may advantageously be omitted.

Furthermore, it may become possible to express $\theta_2$ and $\theta_1$ as functions of the elevation angle $\alpha$ and the longitude angle $\beta$ (equations 15.17):

$$s(\theta_2) = s(\alpha)s(\beta)$$

$$c(\theta_2) = \sqrt{1 - s^2(\alpha)s^2(\beta)}$$

$$s(\theta_1) = \frac{s(\alpha)c(\beta)}{\sqrt{1 - s^2(\alpha)s^2(\beta)}}$$

$$c(\theta_1) = \frac{c(\alpha)}{\sqrt{1 - s^2(\alpha)s^2(\beta)}}$$

**Figure 16** shows a schematic geometrical model of an arm while wearing an exemplary user-wearable orthosis.

Specifically, Figure 16 illustrates several parameters useful for the understanding of an exemplary control of the pulling actuator.

In particular, Figure 16 illustrates the parameter $l_b^*$, corresponding to a cantilever length. Specifically, the parameter $l_b^*$ may correspond to a shortest distance between the second opening of the guiding cavity of the proximal anchor unit and the first rotation axis and/or the second rotation axis.

The parameter $h_b^*$ may correspond to a height of the proximal anchor unit with respect to a center of rotation of the joint, e.g., of the shoulder joint. In particular, the parameter $h_b^*$ may be measured along a vertical model axis extending through the center of rotation of the joint, wherein the vertical model axis may be parallel to and/or identical to the z-axis of the world frame. The height may extend to the exit point of the Bowden sheath, namely the end of the guiding cavity 102.

The parameter $\gamma^*$ may correspond to an angle subtended by the cantilever length $l_b^*$. In particular, parameter $\gamma^*$ may correspond to the angle between a line, which intersects the second opening of the guiding cavity of the proximal anchor unit and the center of rotation of the joint, and the vertical model axis.

The parameter $l_a^*$ may to the length of a straight line extending from the center of rotation of the elbow joint to a projection of the force transfer element connection point of the anchor point element onto the limb axis.

Furthermore, although not shown in Figure 16, the parameter $l_u^*$ may correspond to a shortest distance between the center of rotation of the joint and the center of rotation of the elbow joint, preferably along the limb axis.

The parameter ha may correspond to a height of the force transfer element connection point of the anchor point element with respect to the limb axis.

The parameter $\beta^*$ may correspond to an angle subtended by the height $h_a^*$. In particular, parameter $\beta^*$ may correspond to the angle between a line, which intersects the force transfer element connection point of the anchor point element and the center of rotation of the joint, and the limb axis.

The parameter $k_1^*$ may correspond to a distance between the second opening of the guiding cavity and the center of rotation of the joint.

The parameter $k_2^*$ may correspond to a distance between the force transfer element connection point and the center of rotation of the joint.

The parameter $\alpha(t)$ may correspond to the elevation angle. The parameter $k(t)$ may correspond to the cable length and/or a length of the force transfer element, preferably between the second opening of the guiding cavity and the force transfer element connection point.

Of the above parameters, those parameters marked with an asterisk may represent fixed parameters. Of these fixed parameters, a subset may be determined and/or measured in order to determine the other fixed parameters. For example, the subset may comprise the parameters $l_a^*, l_b^*, l_u^*, h_a^*$, and $h_b^*$, wherein the other fixed parameters may be determined as follows (equations 16.1):

$$\gamma^* = \tan^{-1}\left(\frac{l_b^*}{h_b^*}\right)$$

$$k_1^* = \frac{l_b^*}{s(\gamma^*)}$$

$$\beta^* = \tan^{-1}\left(\frac{h_a^*}{l_u^* - l_a^*}\right)$$

$$k_2^* = \frac{h_a^*}{s(\beta^*)}$$

Specifically, it is therefore possible to derive all of the above parameters by easy measurements, allowing for a simple and efficient adaptation to the respective, individual user.

**Figure 17** shows a graph of a sigmoid interpolation between two values of the adaptive filter parameter $\lambda$ as a function of the elevation angle $\alpha$.

As shown, a maximal value $\lambda_{max}$ is set to 0.98, while a minimal value of $\lambda_{min}$ is set ot 0.63.

The invention is not limited to the herein described and/or shown exemplary embodiments.

**List of Reference Numerals**

| | |
|---|---|
| **1** | User-wearable orthosis |
| **100** | Proximal anchor unit |
| **101** | Force transfer element guiding device |
| **101A; 101B** | Guiding element |
| **102** | Guiding cavity |
| **103** | Guiding element base cavity |
| **104, 105** | Base element |
| **106** | Base cavity |
| **107** | Rotational bearing |
| **108** | Abutment cavity |
| **109** | Bowden sheath abutment device |
| **110, 111, 112, 113** | Fixation bore |
| **114A, 114B** | Fixation screw |
| **200** | Distal anchor unit |
| **201** | Anchor point element |
| **202** | Guidance element |
| **203** | Force transfer element connection point |
| **204** | Movement bas element |
| **205** | Wheel element |
| **206** | Wheel axis |
| **207** | Wheel |
| **208** | Guide rail |
| **209** | Base plate |
| **210, 211** | Guide rail element |
| **212** | Outer surface |
| **213** | Upper rib surface |
| **214** | First rib side surface |
| **215, 216** | Stopping section |
| **300** | Common actuation unit |
| **301** | Pulling actuator |

| 302 | Pulling actuator abutment section |
| 303 | Adjustment actuator |
| 304, 305 | Adjustment actuator abutment section |
| 306 | Housing |
| 307 | Hinged lid |
| 308 | Control unit |
| 309 | Internal space |
| B1 | Bowden sheath |
| B2, B3 | Adjustment Bowden sheath |
| H1 | Shoulder harness element |
| H2 | Torso harness element |
| H3 | Arm harness element |
| L1 | Load cell |
| M | Method |
| M1, M2 | Steps |
| P1 | Pulling force |
| P2 | Adjustment pulling force |
| S1, S2, S3 | IMU sensor |
| T1 | Force transfer element |
| U | User |
| $\alpha$ | Elevation angle |
| $\beta$ | Longitude angle |
| $Z_w$ | z-axis of world frame |
| $Z_u$ | z-axis of limb solidal frame |
| <w> | World frame |
| <u> | Limb solidal frame |
| $Y_u$ | y-axis of limb solidal frame |
| <1>, <2>, <3> | frames |

**Claims**

1. A user-wearable orthosis for assisting a user motion of a joint of a user, comprising:

   a proximal anchor unit mountable on or proximal to the joint;
   a distal anchor unit mountable on a limb attached to the joint, wherein the distal anchor unit comprises an anchor point element moveable at least partially circumferentially around the limb of the user, wherein the proximal anchor unit is connectable to the anchor point element via a force transfer element, wherein the user-wearable orthosis is configured to exert a pulling force on the force transfer element to assist the user motion;
   a movement prediction unit configured to at least partially predict the user motion of the joint; and
   an anchor point element adjustment unit, wherein the anchor point element adjustment unit is configured to control a circumferential position of the anchor point element around the limb based on the at least partially predicted user motion of the joint.

2. The user-wearable orthosis according to claim 1, wherein the distal anchor unit further comprises a guidance element configured to at least partially circumferentially surround the limb of the user,

   wherein the anchor point element is moveable along the guidance element, and
   wherein the anchor point element adjustment unit is configured to control a position of the anchor point element along the guidance element.

3. The user-wearable orthosis according to claim 2, wherein the guidance element is configured to restrict a movement of the anchor point element along an axial direction and/or a radial direction of the limb of the user.

4. The user-wearable orthosis according to one of the preceding claims, wherein the movement prediction unit comprises at least one sensor unit configured to measure joint motion data of the user motion, wherein the movement prediction unit is configured to at least partially predict the user motion of the joint based on the joint motion data; and/or

   wherein the movement prediction unit is configured to at least partially predict the user motion of the joint in and/or along a sagittal plane of the user; and/or

wherein the movement prediction unit is configured to at least partially predict the user motion of the joint in and/or along a frontal plane of the user.

5. The user-wearable orthosis according to one of the preceding claims, wherein the proximal anchor unit comprises:

an anchor base mountable on the user;

a force transfer element guiding device configured to at least partially guide the force transfer element towards the anchor point element; and

a rotational bearing provided between the anchor base and the force transfer element guiding device, wherein the rotational bearing is configured to allow the force transfer element guiding device to substantially freely rotate around a first rotation axis relative to the anchor base in response to a change in the circumferential position of the anchor point element around the limb.

6. The user-wearable orthosis according to claim 5, wherein the user-wearable orthosis further comprises a pulling actuator unit configured to exert the pulling force,

wherein the user-wearable orthosis further comprises the force transfer element, wherein the force transfer element is guided within a Bowden sheath at least partially between the force transfer element guiding device and the pulling actuator,

wherein the proximal anchor unit further comprises:

a Bowden sheath abutment device configured to be connectable to one end of the Bowden sheath,

wherein the Bowden sheath abutment device is connectable to the force transfer element guiding device,

wherein the Bowden sheath abutment device and the force transfer element guiding device are configured to be substantially freely rotatable relative to one another around a second rotation axis in response to the change in the circumferential position of the anchor point element around the limb.

7. The user-wearable orthosis according to claim 6, wherein the first rotation axis is substantially identical to the second rotation axis.

8. The user-wearable orthosis according to one of the preceding claims, wherein the anchor point element adjustment unit comprises an adjustment actuator connectable, via at least one adjustment force transfer element, to the anchor point element,

wherein the adjustment actuator is configured to exert a force on the at least one adjustment force transfer element to control the circumferential position of the anchor point element.

9. The user-wearable orthosis according to claim 8, wherein the adjustment actuator is configured to be worn by the user on a torso or back of the user.

10. The user-wearable orthosis according to claim 8, wherein the adjustment actuator is provided in the distal anchor unit.

11. The user-wearable orthosis according to one of claims 1 to 7, wherein the anchor point element adjustment unit comprises an element adjustment actuator configured to control the circumferential position of the anchor point element,

wherein the element adjustment actuator is provided in the anchor point element.

12. The user-wearable orthosis according to one of the preceding claims, wherein the joint is a shoulder joint of the user, the limb is an arm and/or upper arm of the user, and the user motion of the joint is a lifting of the arm of the user.

13. A method for controlling a user-wearable orthosis for assisting a user motion of a joint of a user, comprising:

at least partially predicting the user motion of the joint; and

controlling a circumferential position of an anchor point element of a distal anchor unit around a limb attached to the joint based on the at least partially predicted user motion of the joint,

wherein the distal anchor unit is mounted on the limb, wherein a proximal anchor unit mounted on or proximal to the joint is connectable to the anchor point element via a force transfer element, wherein the user-wearable orthosis is configured to exert a pulling force on the force transfer element to assist the user motion and

wherein the anchor point element is moveable at least partially circumferentially around the limb of the user.

14. A non-transitory computer readable storage medium containing instructions that, when executed by at least one computing device, cause the computing device to perform the method according to claim 13.

Figure 1

Figure 2

Figure 3

Figure 4

Figure 5

Figure 6

Figure 7

Figure 8

Figure 9A

Figure 9B

Figure 10

Figure 11

Figure 12

M

M1

at least partially predicting the
user motion of the joint

M2

controlling a circumferential position of
an anchor point element of a distal anchor
unit around a limb attached to the joint

Figure 13

# Figure 14

IMU

User's Arm

<3>

<2>

<1>

Figure 15

Figure 16

EMA filter parameter

alpha [°]

lambda

Figure 17

**EUROPEAN SEARCH REPORT**

Application Number

EP 24 16 5234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2020/038218 A1 (INGVAST JOHAN [SE] ET AL) 6 February 2020 (2020-02-06) * paragraphs [0034], [0050]; figure 4 * | 1-14 | INV. A61F5/01 |
| A | US 2016/051388 A1 (GOLDFARB MICHAEL [US] ET AL) 25 February 2016 (2016-02-25) * figure 1 * | 1-14 | |
| A | US 2019/142681 A1 (SEO KEEHONG [KR] ET AL) 16 May 2019 (2019-05-16) * the whole document * | 1-14 | |
| A | US 2016/113831 A1 (HOLLANDER KEVIN [US]) 28 April 2016 (2016-04-28) * paragraphs [0049], [0156] * | 1-14 | |
| A | US 2022/023133 A1 (WOGE SOFIE [SE] ET AL) 27 January 2022 (2022-01-27) * paragraphs [0050] - [0056] * | 1-14 | |
|  |  |  | **TECHNICAL FIELDS SEARCHED (IPC)** |
|  |  |  | A61F B25J |

The present search report has been drawn up for all claims

| Place of search | Date of completion of the search | Examiner |
|---|---|---|
| Munich | 8 August 2024 | Grieb, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**  EP 24 16 5234

This annex lists the patent family members relating to the patent documents cited in the above-mentioned European search report.
The members are as contained in the European Patent Office EDP file on
The European Patent Office is in no way liable for these particulars which are merely given for the purpose of information.

08-08-2024

| Patent document cited in search report | Publication date | Patent family member(s) | | Publication date |
|---|---|---|---|---|
| US 2020038218 A1 | 06-02-2020 | CN | 109890332 A | 14-06-2019 |
| | | EP | 3522838 A1 | 14-08-2019 |
| | | ES | 2809743 T3 | 05-03-2021 |
| | | JP | 7080225 B2 | 03-06-2022 |
| | | JP | 2019528984 A | 17-10-2019 |
| | | KR | 20190065271 A | 11-06-2019 |
| | | SE | 1651308 A1 | 06-04-2018 |
| | | US | 2020038218 A1 | 06-02-2020 |
| | | WO | 2018065459 A1 | 12-04-2018 |
| US 2016051388 A1 | 25-02-2016 | EP | 2991595 A1 | 09-03-2016 |
| | | US | 2016051388 A1 | 25-02-2016 |
| | | US | 2020197208 A1 | 25-06-2020 |
| | | WO | 2014179703 A1 | 06-11-2014 |
| US 2019142681 A1 | 16-05-2019 | CN | 109760015 A | 17-05-2019 |
| | | EP | 3483892 A1 | 15-05-2019 |
| | | EP | 4134965 A1 | 15-02-2023 |
| | | JP | 2019088766 A | 13-06-2019 |
| | | KR | 20190053615 A | 20-05-2019 |
| | | US | 2019142681 A1 | 16-05-2019 |
| US 2016113831 A1 | 28-04-2016 | NONE | | |
| US 2022023133 A1 | 27-01-2022 | EP | 3893814 A1 | 20-10-2021 |
| | | JP | 7450621 B2 | 15-03-2024 |
| | | JP | 2022513826 A | 09-02-2022 |
| | | SE | 1851567 A1 | 13-06-2020 |
| | | US | 2022023133 A1 | 27-01-2022 |
| | | WO | 2020122792 A1 | 18-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. XILOYANNIS et al.** Soft Robotic Suits: State of the Art, Core Technologies, and Open Challenges. *IEEE Transactions on Robotics*, June 2022, vol. 38 (3)